# FASCICULE DE BREVET EUROPEEN

(11) **EP 3 288 575 B1**
(45) Date de publication et mention de la délivrance du brevet: **11.09.2019**
(21) Numéro de dépôt: 16725186.7
(22) Date de dépôt: 29.04.2016
(51) Int. Cl.: A61K 38/02, A61K 31/435, A61K 35/36, A61K 8/64, A61K 8/98, A61Q 19/08, A61Q 17/04, A61P 35/00, A61P 39/06, A61P 27/02

(54) **COMPLEXE PROTÉOMÉLANIQUE BIOASSIMILABLE, PRÉPARATION ET UTILISATIONS**
BIOASSIMILIERBARER PROTEIN-MELANIN-KOMPLEX, HERSTELLUNG UND VERWENDUNGEN
BIOASSIMILABLE PROTEIN-MELANIN COMPLEX, PREPARATION AND USES

(30) Priorité: 30.04.2015 FR 1553920
(43) Date de publication de la demande: 07.03.2018
(73) Titulaire: Carlini, Rosanna, 13600 La Ciotat (FR)
(72) Inventeur: BLANCHARDIE, Olivier, 83130 La Garde (FR); VELLA, Jean-Sébastien, 13011 Marseille (FR)
(74) Mandataire: Santarelli
(86) Numéro de dépôt international: PCT/FR2016/051010
(87) Numéro de publication internationale: WO 2016/174367

(56) Documents cités:
- US-A1- 2003 096 735
- US-B1- 6 315 988
- LIU YAN ET AL: "Comparison of the structural and physical properties of human hair eumelanin following enzymatic or acid/base extraction", PIGMENT CELL RESEARCH, MUNKSGAARD INTERNATIONAL PUBLISHERS, CAMBRIDGE, MA, DK, vol. 16, no. 4, 1 août 2003 (2003-08-01), pages 355-365, XP009186851, ISSN: 0893-5785
- GOLOUNIN A V: "Production of melanin useful in cosmetic and cancer- from human hair, horse hair or wool by alkali extraction an boiling", WPI WORLD PATENT INF, 1 janvier 1998 (1998-01-01), XP002120229,

## Description

La présente invention concerne un complexe protéomélanique bioassimilable bioactif, comprenant un extrait protéique, avantageusement au moins partiellement soluble, et de la mélanine. Le complexe selon, l'invention présente en outre une bonne assimilation ultérieure dans le corps humain. L'invention concerne également un procédé de préparation dudit complexe protéomélanique bioassimilable selon l'invention, l'utilisation dudit complexe ainsi que des compositions comprenant au moins ledit complexe.

Les mélanines sont des polymères biologiques complexes que l'on trouve chez les humains, les animaux, les plantes, les bactéries, les champignons et les protistes.

Chez l'homme, les mélanines sont présentes dans la peau, les cheveux, des parties du cerveau et des parties de l'oeil et les oreilles.

Les mélanines biologiques sont des macromolécules produites pour la plupart par les mélanocytes, par addition ou condensation de monomères formés à partir de la tyrosine (eumélanine) ou de la tyrosine et de la cystéine (phéomélanine), avec le concours de la tyrosinase (Plonka, P.M. and Grabacka, M. ; Melanin synthesis in microorganisms- biotechnological and medical aspects Acta Biochimica Polonica 2006 53: 429-443).

Il existe également des mélanines artificielles telles les polyacétylènes, les polyanilines, et les polypyrroles de couleur noire ou brune, et leurs copolymères ; elles ont une application industrielle et/ou biotechnologique.

Dans le présent texte et sous réserve d'indication particulière les termes "mélanine" ou "mélanines" pourront être utilisés indifféremment pour désigner des mélanines naturelles et/ou artificielles, de quelque origine que ce soit, seules ou en mélange, qu'elles soient de l'eumélanine, de la phéomélanine, ou un mélange des 2 en toutes proportions ou encore des produits qui intermédiaires de synthèse, de leurs dégradations ainsi que leurs formes éventuellement modifiées.

Dans la structure des mélanines, les radicaux libres existent en équilibre avec les groupes non-radicaux.

Les mélanines sont très résistantes à la pourriture et à la biodégradation.

Les mélanines possèdent des propriétés antioxydantes fortes et peuvent protéger contre les attaques des enzymes lytiques.

Les mélanines présentent des propriétés radioprotectrices du fait de leur capacité à absorber les radionucléides de manière efficace.

Les mélanines peuvent contribuer à la réduction de l'excès de métaux lourds dans le corps humain.

Les mélanines sont réputées pour protéger efficacement les organismes vivants du rayonnement ultraviolet (UV).

Le potentiel thérapeutique des mélanines pour des applications en médecine, en pharmacologie, en cosmétique, sous toutes formes compatibles telles des compositions pharmaceutiques et/ou cosmétiques, ou encore des supplémentations nutritionnelles, est bien documenté (Plonka, P.M. et Grabacka M., *opus citatum).*

Les mélanines jouent un rôle important de photoprotection, particulièrement de la peau humaine, contre les effets aigus de l'exposition aux UV (par exemple les coups de soleil), comme les risques potentiels à long terme des changements actiniques (rides et vieillissement prématuré de la peau) et des états précancéreux ou malins (kératose solaire, carcinomes basocellulaires et/ou squameux des carcinomes des cellules, tumeurs malignes).

Les mélanines (principalement l'eumélanine) sont présentes dans l'épiderme sous une forme particulaire, le mélanosome, et sous une forme amorphe, mélanines colloïdales, et offrent au travers de ces formes une protection aux cellules.

En plus de la peau, les mélanines sont également présentes dans l'épithélium pigmentaire rétinien (EPR) de l'oeil humain. Les mélanines de l'EPR, principalement l'eumélanine, joue un rôle photoprotecteur par absorption du rayonnement et en piégeant les radicaux libres réactifs et les espèces réactives de l'oxygène (ROS).

Un rôle phototoxique des mélanines de l'EPR, en particulier dans les cellules âgées, y compris la photogénération accrue de ROS tels que les anions superoxydes et les radicaux hydroxyles qui sont impliqués dans la mort des cellules de l'EPR a été également décrit.

La chimie des mélanines est d'un intérêt particulier parce que la mort de cellules de l'EPR est une caractéristique majeure de la pathogenèse de la dégénérescence maculaire liée à l'âge (DMLA), principale cause de cécité dans la population humaine de plus de 60 ans dans le monde développé (Brandon-Luke, L. et al. Time Resolved Detection of Melanin Free Radicals Quenching Reactive Oxygen Species J. Am. Chem. Soc. 2005 127, 11220-1221).

Afin d'identifier les moyens d'exploiter davantage les fonctionnalités des mélanines et de permettre à leurs applications biotechnologiques potentielles d'émerger, l'accent a été mis sur la solubilisation aqueuse des mélanines.

Les mélanines solubles peuvent être produites par synthèse ou isolées à partir de sources naturelles (US 5,216,116; US 5,218,079; US 5,225,435; US 5,227,459; US5,384,116; US 5,574125; US6,315,988; US 5,814,495 US 6,576,268).

Bien qu'une quantité importante de travaux aient été réalisés sur la synthèse de mélanines synthétiques, la production de mélanine à partir de bactéries ou de sources végétales comme les légumes et les fruits, la production de mélanines de mammifères est restée largement inexploitée parce que les mélanines de mammifères sont très insolubles et exigent des traitements sévères tels que l'ébullition en milieu basique fort, ou l'utilisation d'oxydants forts tels que le peroxyde d'hydrogène, ce qui entraîne des dommages et dénature les mélanines produites ainsi que les complexes protéiques entourant lesdites mélanines.

De nombreux travaux ont été réalisés sur les mélanines naturelles obtenues à partir de végétaux ou de sources bactériennes rendues par la suite solubles dans l'eau. Ces travaux ont conduit à de multiples applications topiques des mélanines en cosmétique et dermatologie ce qui a permis de constater effectivement une certaine absorption cutanée des mélanines.

L'intérêt pour les rôles biologiques et thérapeutiques des mélanines sur le corps humain est croissant, mais peu de travaux ont été réellement réalisés dans le but d'obtenir une mélanine réellement bioassimilable par le corps humain, particulièrement lorsque les mélanines sont d'origine animale particulièrement de mammifères.

Il existe donc un besoin réel en mélanines bioassimilables ou rendues bioassimilables, particulièrement en mélanines bioassimilables ou rendues bioassimilables d'origine animale, plus particulièrement d'origine mammifère, lesdites mélanines ayant de plus avantageusement conservées leurs propriétés, particulièrement biologiques, intrinsèques, donc caractérisables comme des mélanines actives ou bioactives. C'est un des buts de la présente invention que de proposer une mélanine, avantageusement d'origine animale, mammifère ou aviaire, préférentiellement mammifère, bioassimilable ou rendue bioassimilable, de préparation aisée et présentant des propriétés utilisables, tant chez l'être humain que chez les animaux, dans les domaines cosmétique, dermatologique, pharmaceutique, ou alimentaire, parce qu'ayant conservée ses propriétés, particulièrement biologiques, intrinsèques.

Les travaux de la Demanderesse l'ont en effet conduite à mettre au point un complexe protéomélanique présentant la propriété d'être bioassimilable et de présenter une mélanine ayant conservée ses propriétés, particulièrement biologiques, intrinsèques et capables de les exprimer une fois introduite dans l'organisme du receveur (mélanine bioactive). Ainsi il sera possible par ailleurs dans le présent texte de qualifier le complexe protéomélanique objet de l'invention de bioassimilable et/ou de bioactif.

Selon la présente invention on entend par complexe une association de molécules, plus ou moins intimement liées entre-elles.

Dans le cas particulier de la présente invention ledit complexe protéomélanique devra s'entendre comme pouvant au moins comprendre un extrait protéique et de la mélanine plus ou moins intimement liés.

Par bioassimilable on entend, selon la présente invention, la capacité d'une molécule ou d'une composition, à passer les barrières biologiques, telles la peau dont le cuir chevelu, ou encore la barrière gastro-intestinale, et à se retrouver disponible dans la circulation, avantageusement sans être dégradée, partiellement ou totalement, ou éliminée.

L'invention a ainsi pour objet premier un complexe protéomélanique bioassimilable, pouvant comprendre au moins un extrait protéique, soluble ou partiellement soluble et de la mélanine, ledit complexe protéomélanique pouvant être avantageusement bioactif et ledit extrait protéique comprenant au moins un résidu cystéine ou au moins un résidu tyrosine ou au moins un résidu cystéine et un résidu tyrosine caractérisé en ce que les protéines et/ou les fragments de protéines dudit extrait protéique ont été rendus solubles par greffage d'au moins un groupement sulfite sur au moins un atome de soufre d'au moins une cystéine desdites protéines et/ou desdits fragments de protéines dudit extrait protéique.

Par extrait protéique on entend selon l'invention que ledit complexe protéomélanique selon l'invention pourra comprendre des protéines et/ou des fragments de protéines (peptides) issues de la source protéique ou de la source mélanoprotéique retenue. Par source mélanoprotéique on entend ici une seule source qui contient à la fois des protéines et de la mélanine.

Par extrait protéique soluble ou partiellement soluble on entend selon l'invention que les protéines et/ou les fragments de protéines (peptides) présents dans ledit extrait ont été rendus solubles par toute technique de solubilisation des protéines et/ou des fragments de protéines (peptides) connue de l'homme du métier comme par exemple l'hydrolyse ou une modification chimique. A titre de modification chimique on pourra citer à titre d'exemple le greffage de groupements polaires sur lesdites protéines et/ou fragments de protéines (peptides), particulièrement sur les cystéines desdites protéines et/ou desdits fragments de protéines (peptides) très particulièrement sur l'atome de soufre desdites cystéines.

Par cystéine on entend selon l'invention la cystéine en tant que telle, libre ou impliquée dans une liaison peptidique, ou encore la cystine.

Selon l'invention les protéines et/ou les fragments de protéines (peptides) sont rendus solubles par greffage d'au moins un groupement sulfite sur l'atome de soufre d' au moins une cystéine desdites protéines et/ou desdits fragments de protéines (peptides). L'extrait protéique soluble ou partiellement soluble présent dans ledit complexe protéomélanique comprend au moins une cystéine porteuse d'un groupement sulfite sur son atome de soufre, par ailleurs dénommée dans le présent texte cystéine S-sulfonée ou S-sulfocystéine.

Avantageusement selon l'invention ledit extrait protéique soluble ou partiellement soluble présent dans ledit complexe protéomélanique peut comprendre entre une et toutes ses cystéines sous la forme de cystéines S-sulfonées, très avantageusement entre un tiers et toutes, encore plus préférentiellement entre la moitié et toutes de ses cystéines sous la forme de cystéines S-sulfonées. Selon une forme très préférée de l'invention ledit extrait protéique soluble ou partiellement soluble présent dans ledit complexe protéomélanique pourra comprendre 100% de ses cystéines sous la forme de cystéines S-sulfonées.

Selon l'invention, ledit extrait protéique pourra être soluble dans l'eau de 0,1 à 99 %, préférentiellement de 0,1 à 75%, très préférentiellement de 0,1 à 50%.

La solubilité peut être mesurée à l'aide d'un turbidimètre HI 83703 de chez HANNA Instrument (conçu en accord avec les normes ISO 7027 du standard international FTU (Formazine Turbitity Unit) équivalent au NTU (Nephelometric Turbidity Unit) et destiné à mesurer très précisément la turbidité. Plus la turbidité est faible, plus l'échantillon est soluble ; plus la turbidité est élevée, plus il y a de microparticules et de corps insolubles dans le milieu.

A une concentration comprise entre 3 et 10% dans l'eau, ledit extrait protéique ou protéomélanique d'intérêt présente une turbidité extrêmement faible voire nulle, de 0.00 à 50 NTU, Ledit extrait protéique ou protéomélanique d'intérêt peut être soluble, de telle sorte qu'il peut passer à travers des filtres de porosité 5 à 100 µm sans être retenu. L'extrait soluble d'intérêt peut représenter plus de 50% de rendement en rapport à la masse protéique ou mélanoprotéique issue de la matière première utilisée, déterminée sur masse sèche avant et après filtration.

Selon l'invention, ledit complexe protéomélanique, pourra comprendre de 0,01% à 99,99% d'extrait protéique, préférentiellement entre 1% et 95%, très préférentiellement entre 30% et 95%, encore plus préférentiellement entre 50% et 95%.

Selon l'invention, ledit complexe protéomélanique, pourra comprendre de 0,01% à 99,99% de mélanine, préférentiellement entre 0,5% et 20%, très préférentiellement entre 1% et 15%.

Selon l'invention, ledit extrait protéique pourra comprendre entre 0,1% et 100% de fragments de protéines (peptides), préférentiellement entre 30% et 100%, très préférentiellement entre 55% et 100% de fragments de protéines (peptides).

Selon l'invention, lesdits fragments de protéines (peptides) pourront avoir une longueur comprise entre 2 et 1000 acides aminés, préférentiellement entre 2 et 500 acides aminés, très préférentiellement entre 2 et 100 acides aminés.

Selon une variante de l'invention ledit extrait protéique et ladite mélanine peuvent être issus de sources protéiques et mélaniques différentes ou identiques, prises séparément.

Selon une autre variante de l'invention, préférée, ledit extrait protéique et ladite mélanine peuvent être issus d'une même source mélanoprotéique, avantageusement utilisée seule.

On notera que selon l'invention, tant la source de mélanine que la source protéique et que la source mélanoprotéique peuvent être uniques ou un mélange de différentes source de mélanine, protéique ou mélanoprotéiques, en toutes proportions.

Ainsi selon l'invention la mélanine et/ou l'extrait protéique peuvent être issus
- d'une source de mélanine unique et d'une source protéique unique ;
- d'un mélange de sources de mélanine et d'une source protéique unique ;
- d'un mélange de sources de mélanine et d'une source protéique unique et d'une source mélanoprotéique unique ;
- d'un mélange de sources de mélanine et d'une source protéique unique et d'un mélange de sources mélanoprotéiques ;
- d'une source de mélanine unique et d'un mélange de sources protéiques ;
- d'une source de mélanine unique et d'un mélange de sources protéiques et d'une source mélanoprotéique unique ;
- d'une source de mélanine unique et d'un mélange de sources protéiques et d'un mélange de sources protéomélaniques ;
- d'un mélange de sources de mélanine et d'un mélange de sources protéiques ;
- d'un mélange de sources de mélanine et d'un mélange de sources protéiques et d'une source mélanoprotéique unique ;
- d'un mélange de sources de mélanine et d'un mélange de sources protéiques et d'un mélange de sources mélanoprotéiques ;
- d'une source mélanoprotéique unique ;
- d'une source mélanoprotéique unique et d'une source de mélanine unique ;
- d'une source mélanoprotéique unique et d'une source protéique unique ;
- d'un mélange de sources mélanoprotéiques et d'une source de mélanine unique ;
- d'un mélange de sources mélanoprotéiques et d'une source protéique unique.

Selon l'invention, la source de mélanine pourra être de synthèse, de semi -synthèse ou obtenue par extraction d'une source naturelle. On utilisera de préférence une mélanine d'extraction naturelle. La source de mélanine naturelle peut être d'origine animale, y compris humaine, végétale, fongique, ou micro-organique, préférentiellement d'origine animale, y compris humaine, très préférentiellement d'origine mammifère ou aviaire, encore plus préférentiellement d'origine mammifère.

Ainsi comme source de mélanine naturelle utilisable selon l'invention, on peut citer à titre d'exemple la mélanine issue de laine, de poils, de cheveux, de griffes, de cornes ou encore de plumes, de plantes, de fruits, d'encre de céphalopodes, de bactéries ou de sources synthétiques. Préférentiellement, la source de mélanine pourra être de la laine comme celle de moutons, de mouflons, de chèvres, de chamois, de takin, de bouquetins, de yanghirs, de thars, de jharal, de serows, de goral, de boeuf musqué, d'urial, de bharal, d'isard, de lapins, de lièvres, de pikas, de lama, d'alpaga, de guanaco, de vigogne, de chameau, de dromadaire, de yack ou de plumes comme celle de pie, de corbeau ou de merle. Une laine préférée peut être de la laine de mouton, particulièrement de mouton noir (comme par exemple ceux des races "mouton d'Ouessant", "Noire du Velay", "Nez-noir du Valais", "mouton Noir de Thibar", "Black Welsh Mountain Sheep", "Balwen", "Zwartbles" ou encore "Hebridean"), très précisément de la laine de mouton de la race "Noire du Velay".

L'homme du métier aura compris que dans le présent texte le terme "mouton" (*Ovis aries*) est utilisé pour désigner un mammifère domestique herbivore de la famille des bovidés, de la sous-famille des Caprinés et du genre *Ovis*. Le terme couvre aussi bien l'animal jeune (agneau/agnelle), la femelle (brebis) que le mâle (bélier), châtré ou non.

Des exemples de dérivés de mélanine synthétique utilisables sont décrits dans les demande de brevet US 5,618,519, US 5,384,116, et US 5,227,459. Des exemples de dérivés de mélanine soluble utilisables sont décrits dans les demande de brevet US 5,744,125, US 5,225,435, US 5,218,079, et US 5,216,116. Des exemples de dérivés de mélanine soluble commerciaux utilisables sont par exemple la Melasyn-100™ de San-mar laboratories, Inc. (Elmsford, N.Y. USA) et la MelanZe™ de Zylepsis (Ashford, Kent, Grande Bretagne).

Selon l'invention, ledit extrait protéique peut provenir de toute source protéique naturelle ou synthétique, préférentiellement naturelle. Ladite source protéique naturelle peut être de toute origine et sous toute forme. Préférentiellement elle pourra être d'origine animale ou végétale, fongique, ou microorganique, avantageusement d'origine animale ou végétale, préférentiellement animale, très préférentiellement d'origine mammifère ou aviaire, encore plus préférentiellement d'origine mammifère.

Selon une forme préférée de l'invention la source protéique pourra être une source animale, préférentiellement une source protéique comprenant de la kératine comme par exemple les phanères de nombreux animaux, avantageusement de mammifères y compris l'Homme, parmi lesquels on peut citer les poils, les cheveux, les vibrisses, la laine, les plumes, les cornes, les ongles, les griffes, les sabots, les becs, les écailles.

On comprend de ce qui précède que lorsque selon l'invention on utilise une source mélanoprotéique celle-ci peut être de toute origine pour peu qu'elle contienne à la fois des protéines et de la mélanine. Selon l'invention la source mélanoprotéique pourra être d'origine animale, végétale, fongique, ou microorganique, avantageusement d'origine animale, avantageusement de mammifères y compris l'Homme, très avantageusement les phanères, parmi lesquels on peut citer les poils, les cheveux, les vibrisses, la laine, les plumes, les cornes, les ongles, les griffes, les sabots, les becs, les écailles.

Préférentiellement selon l'invention, ladite source mélanoprotéique pourra être de la laine, comme celle de moutons, de mouflons, de chèvres, de chamois, de takin, de bouquetins, de yanghirs, de thars, de jharal, de serows, de goral, de boeuf musqué, d'urial, de bharal, d'isard, de lapins, de lièvres, de pikas, de lama, d'alpaga, de guanaco, de vigogne, de chameau, de dromadaire, de yack ou de plumes comme celle de pie, de corbeau ou de merle. Une laine préférée peut être de la laine de mouton, particulièrement de mouton noir (comme par exemple ceux des races "mouton d'Ouessant", "Noire du Velay", "Nez-noir du Valais", "mouton Noir de Thibar", "Black Welsh Mountain Sheep", "Balwen", "Zwartbles" ou encore "Hebridean"), très précisément de la laine de mouton de la race "Noire du Velay".

Une forme préférée du complexe protéomélanique bioassimilable selon l'invention pourra être un complexe dont l'extrait protéique et la mélanine pourront être issus d'une seule et unique source mélanoprotéique, avantageusement de la laine noire, très avantageusement de la laine noire de mouton, particulièrement de la laine noire de mouton de la race "Noire du Velay", pouvant comprendre de 0,01% à 99,99% d'extrait protéique, préférentiellement entre 1% et 95%, très préférentiellement entre 30% et 95%, encore plus préférentiellement entre 50% et 95%, comprenant lui-même de 0,1% à 100% de fragments de protéines (peptides), préférentiellement entre 30% et 100%, très préférentiellement entre 55% et 100% dont la longueur pourra être comprise entre 2 et 1000 acides aminés, préférentiellement entre 2 et 500 acides aminés, très préférentiellement entre 2 et 100 acides aminés, et 0,01% à 99,99% de mélanine, préférentiellement entre 0,5% et 20%, très préférentiellement entre 1% et 15%, et pouvant comprendre entre 10 % et 100% de ses cystéines sous la forme de cystéines S-sulfonées, très avantageusement entre 30% et 100%, encore plus préférentiellement entre 50% et 100% de ses cystéines sous la forme de cystéines S-sulfonées, très préférentiellement ledit complexe protéomélanique pourra comprendre 100% de ses cystéines sous la forme de cystéines S-sulfonées.

L'invention a également pour objet un procédé de préparation dudit complexe protéomélanique bioassimilable selon l'invention, selon lequel dans une première étape on rend la partie protéique de ladite source protéique ou mélanoprotéique solubilisable, avantageusement hydrosolubilisable, dans une deuxième étape on fractionne la partie protéique du mélange obtenu à la première étape en peptides, pour obtenir un mélange comprenant le complexe protéomélanique recherché.

En fait selon une première forme de réalisation dudit procédé selon l'invention, si les sources protéiques et mélaniques sont physiquement différentes l'une de l'autre selon le procédé selon l'invention, dans une première étape on rend la partie protéique de ladite source protéique solubilisable, avantageusement hydrosolubilisable, dans une deuxième étape on fractionne la partie protéique du mélange obtenu à la première étape en peptides, dans une troisième étape on introduit la mélanine dans le mélange obtenu à la deuxième étape, pour obtenir un mélange comprenant le complexe protéomélanique recherché.

Il est notable que selon ce procédé l'introduction de la mélanine peut aussi bien avoir lieu avant la première étape destinée à rendre les protéines solubilisables qui pourra alors avoir lieu sur un mélange source protéique / source mélanique physiquement différentes, après la première étape donc avant la deuxième étape de fractionnement des protéines qui alors pourra avoir lieu sur un mélange source protéique rendue solubilisable / source mélanique, ou après la deuxième étape par ajout de la mélanine sur le mélange source protéique rendue solubilisable et fractionné. Préférentiellement selon l'invention, dans cette forme de réalisation du procédé, l'introduction de la mélanine pourra être faite après la deuxième étape.

Selon une deuxième forme de réalisation dudit procédé selon l'invention, si les sources protéiques et mélaniques sont physiquement identiques (source mélanoprotéique) selon le procédé selon l'invention dans une première étape on rend la partie protéique de ladite source mélanoprotéique solubilisable, avantageusement hydrosolubilisable, dans une deuxième étape on fractionne la partie protéique du mélange obtenu à la première étape en peptides, pour obtenir un mélange comprenant le complexe protéomélanique recherché. On comprend que dans cette forme de réalisation du procédé la mélanine est présente dès la première étape.

En elle-même chaque étape du procédé selon l'invention peut être réalisée avec des techniques parfaitement connues de l'homme du métier. L'originalité du procédé mis au point par les inventeurs réside dans la réunion des différentes étapes qui permet d'aboutir à un complexe protéomélanique bioassimilable, éventuellement soluble selon les conditions de pH et qui plus est présente des propriétés utilisables dans les domaines cosmétique, dermatologique, pharmaceutique ou alimentaire.

Selon l'invention ledit mélange obtenu à la dernière étape, comprenant ledit complexe protéomélanique selon l'invention, peut être utilisé directement ou subir toute étape envisageable à sa transformation en un produit de forme plus adaptée pour ses utilisations postérieures.

Selon l'invention, les sources de protéines et de mélanines peuvent se présenter sous toute forme compatible avec la mise en oeuvre du procédé selon l'invention. Par exemple les protéines peuvent être sous forme liquides, solides ou semi solides [par exemple collagènes, hydrolysats de collagène ou gélatines, protéines de lait, caséines, protéines végétales (de soja, blé, riz... etc) sous différentes formes (granulés, solutions, bâtonnés, paillettes, revalorisation de déchets alimentaires...etc)]. La mélanine peut être sous forme liquide, solide, semi solide, (comme par exemple l'encre de seiche en poudre).

Selon l'invention, la première étape destinée à rendre la partie protéique du mélange solubilisable, avantageusement hydrosolubilisable, peut être réalisée par toutes méthodes connues de l'homme du métier comme par exemple le greffage de groupements, avantageusement polaires (hydrophiles donc solubles dans l'eau), pouvant rendre lesdites protéines ou lesdits peptides solubles, sur lesdits protéines ou peptides ou par rupture des liaisons chimiques entre les chaines protéiques ou peptidiques comme par exemple la réduction des ponts disulfure. On pourra citer, par exemple, les réactions de greffage de groupements phosphates, sulfites ou sulfates pouvant être catalysées chimiquement ou enzymatiquement, qui peuvent être réalisées par toutes méthodes connues de l'art antérieur comme par exemple la phosphorylation, la sulfatation, ou encore la sulfitolyse oxydative, étant entendu que la partie protéique du mélange peut être soumise à l'une ou plusieurs de ces réactions de greffage consécutives ou associées. Préférentiellement selon l'invention on pourra utiliser la sulfitolyse oxydative qui présente la double particularité d'aboutir au greffage de sulfites, groupement polaires, sur les chaines latérales des résidus cystéine et également de couper les ponts disulfures libérant les chaines protéiques les unes des autres et favorisant leur solvatation dans l'eau [Crewther W. G. et coll., The chemistry of keratins in Advances in protein chemistry, Academic Press, vol. 20 ; 1965, pages 191-346 ; Otto Lindner, Lars Rodefeld, Benzenesulfonic Acids and Their Derivatives, Wiley-VCH Verlag GmbH & Co, coll. « Ullmann's Encyclopedia of Industrial Chemistry », 15 septembre 2000 ; FR2521571 (19 août 1983) ; US3,644,084 (1er décembre 1971). Avantageusement au cours de cette réaction le ou les groupements polaires, avantageusement le ou les sulfites pourront être greffés sur les cystéines desdites protéines et/ou desdits fragments de protéines (peptides), très particulièrement sur l'atome de soufre desdites cystéines.

Selon l'invention, la deuxième étape d'hydrolyse peut être réalisée par toutes méthodes d'hydrolyse connues de l'art antérieur comme par exemple l'hydrolyse acide, l'hydrolyse basique ou encore l'hydrolyse enzymatique, préférentiellement l'hydrolyse enzymatique. Il est notable que cette étape peut également être réalisée par oxydation partielle en utilisant de l'acide peracétique, du peroxyde d'hydrogène ou un équivalent, dans les conditions connues de l'homme du métier. Préférentiellement selon l'invention la deuxième étape du procédé pourra être réalisée par hydrolyse enzymatique selon les méthodes décrites dans la littérature relative aux hydrolysats protéiques enzymatiques comme par exemple Industrial Enzymes: Structure, Function and Applications (2007, Julio Polaina, Andrew P. MacCabe Ed., published by Springer).

Selon une variante de l'invention les étapes 1 et 2 peuvent être interverties.

Selon une variante de l'invention, une étape d'inactivation enzymatique peut être éventuellement réalisée permettant de garantir l'absence de toute activité protéolytique résiduelle. Cette étape d'inactivation, optionnelle, peut être réalisée juste après l'étape 2, ou à la suite ou pendant toute étape ultérieure comme décrit ci-dessous.

Selon une autre variante de l'invention le mélange obtenu à la dernière étape du procédé ci-dessus décrit peut alors subir toutes les étapes de transformation envisageables pour en obtenir une forme purifiée. Par "purifié" on entend ici que le mélange obtenu en fin du procédé tel que décrit ci-dessus a subi au moins une étape supplémentaire visant à enrichir le mélange obtenu en complexe protéomélanique selon l'invention. A cet égard on peut envisager une étape de séparation des phases liquide et solide, par exemple par filtration, frontale ou tangentielle comme par exemple la filtration sous vide ou par passage sur de la laine lavée ou bien par gravité comme par exemple la sédimentation, la flottation ou la centrifugation.

On peut envisager également une concentration ou une ou des étapes de purification, avantageusement destinées à éliminer, totalement ou partiellement, les minéraux et/ou les résidus réactionnels éventuellement présents dans le mélange tels que par exemple les sulfates, l'éventuel excès de cuivre ou encore de sodium. Les sulfites libres pourront éventuellement être transformés en sulfate avant leur élimination par oxydation avec de l'oxygène actif, sous quelque forme qu'il soit.

A cet égard toutes les méthodes connues de l'art antérieur peuvent être utilisées. On citera à titre d'exemple la séparation par chromatographie sélective sur colonne par exemple de type ionique. Avantageusement les composants, réactifs et/ou solvant éventuellement utilisés pourront être de qualité alimentaire. On peut également citer la séparation membranaire ou par précipitation sélective.

Ceci peut être réalisé par l'utilisation d'agents chélatants, tels que l'acide éthylènediamine tétraacétique, ou les résines échangeuses d'ions, tels que celles contenant des groupes fonctionnels iminodiacétiques, et l'utilisation de la précipitation isoélectrique pour séparer les types de protéines. L'ultrafiltration peut être utilisée à plusieurs étapes dans le processus afin d'améliorer l'efficacité de l'élimination de réactif ou de séparation des protéines.

Une fois purifiés, le produit obtenu en fin de procédé qui contient le complexe protéomélanique bioassimilable selon l'invention, avantageusement le complexe protéomélanique bioassimilable pur, peut être séché par toutes méthodes connues de l'homme du métier comme par exemple le séchage par lit fluidisé, la pulvérisation, la lyophilisation ou encore par atomisation. Il est également possible si souhaité que des étapes supplémentaires de type broyage, mixage ou autre, soient réalisées pour obtenir une granulométrie spécifique aux utilisations postérieures envisagées.

L'homme du métier comprend que c'est au cours de la dernière étape que le complexe protéomélanique selon l'invention se constitue entre la fraction peptidique soluble et la mélanine de sorte que les deux fractions soient parfaitement homogénéisées et liées.

En effet, à ce stade du procédé, la fraction peptidique peut être liée par des liaisons faibles et ioniques à la fraction mélanique rendue hydrosoluble dans le milieu réactionnel dès lors que celle-ci se trouve dans des conditions de pH et température adéquate, lui permettant d'être liée aux peptides. A ce stade l'homme du métier saura sans difficulté adapter) les conditions de pH et/ou de température du milieu pour obtenir le complexe protéomélanique selon l'invention sous la forme qu'il désire. A titre d'exemple il est possible de se placer dans une gamme de pH différente de celle du pHi des protéines/peptides solubles assurant la présence d'une charge sur les peptides en question facilitant leur interaction avec la mélanine. Dans cette option on pourra se placer à un pH supérieur au pHi sur une plage de 5 à 11, idéalement sur une plage de 6 à 8.

Par exemple, si à la première étape du procédé, la sulfitolyse oxydative est utilisée pour rendre la fraction protéique solubilisable alors le milieu réactionnel contiendra du cuivre sous la forme de complexe cuproammonium. Ce milieu réactionnel se retrouvant à l'issu du procédé ledit complexe cuproammonium sera toujours présent à l'issu de l'étape d'hydrolyse. Dans les conditions opératoires, les ions cuivre peuvent établir des interactions ioniques sur les fractions mélanique et protéique participant à l'obtention d'un complexe protéomélanique.

Par ailleurs, pour éviter toute dénaturation du complexe protéomélanique (précipitation, altération chimique...) l'ensemble des opérations optionnelles subséquentes réalisées dans le but de purifier et concentrer le complexe pourront être conduites à une température ne permettant pas ces dégradations. Ainsi la température pour ces opérations pourra ne pas excéder 100°C, préférentiellement 90°C, très préférentiellement 75°C, même pendant un temps très court (de quelques secondes à plusieurs heures).

Le procédé selon l'invention est un procédé doux qui permet d'obtenir un complexe protéomélanique, avantageusement un complexe kératino/mélanique sans dégradation de la mélanine et qui permet d'obtenir un complexe protéomélanique bioassimilable présentant des propriétés, avantageusement biologiques intéressantes qui permettent d'envisager son utilisation dans de nombreux domaines et sous de nombreuses formes galéniques. Parmi les propriétés remarquables du complexe protéomélanique bioassimilable selon l'invention on note sa bioassimilation remarquable, permettant d'envisager la préparation de formulation comprenant ledit complexe protéomélanique bioassimilable adaptées à une administration par voie orale ou par voie d'application topique sur la peau.

Outre une bioassimilation remarquable, les études conduites par les inventeurs ont permis de mettre en évidence que le complexe protéomélanique bioassimilable selon l'invention peut être apte à stimuler la mélanogénèse, particulièrement au niveau de la peau et/ou des cheveux et/ou des yeux, mais également dans le cerveau et dans le canal intérieur de l'oreille. Cette propriété peut permettre de favoriser l'augmentation de la pigmentation de la peau à la fois avec et sans exposition aux rayonnements Ultra-Violets, ainsi d'ailleurs qu'une régénération de la coloration des phanères particulièrement des poils, des cheveux ou encore de la laine et qu'une régénération de la pigmentation de l'oeil. Ces propriétés peuvent être utilisées chez les animaux, préférentiellement les mammifères, particulièrement l'homme.

Ainsi le complexe protéomélanique bioassimilable selon l'invention peut être utilisé pour introduire, par voie non chirurgicale, dans l'organisme de la mélanine exogène, particulièrement de la mélanine exogène ayant conservé ses propriétés intrinsèques.

Le complexe protéomélanique bioassimilable selon l'invention peut être utilisé pour la mise en application de toutes les propriétés connues de la mélanine.

Ainsi l'invention a aussi pour objet le complexe protéomélanique bioassimilable selon l'invention pour stimuler la mélanogénèse, particulièrement dans la peau, dans les phanères, dans les cheveux, dans les poils, dans la laine, dans les yeux, dans le cerveau et/ou dans le canal intérieur de l'oreille, préférentiellement dans les cheveux, les poils, la laine et/ou les yeux.

L'invention a encore pour objet le complexe protéomélanique bioassimilable selon l'invention pour piéger les radicaux libres, avantageusement ainsi combattre leurs effets néfastes comme le vieillissement tissulaire, en particulier l'apparition des rides et des ridules de la peau.

L'invention a aussi pour objet le complexe protéomélanique bioassimilable selon l'invention pour augmenter la régénération cellulaire, l'élasticité et/ou l'hydratation de la peau, pour renforcer la cohésion cellulaire, particulièrement dans la peau et/ou les phanères.

Cette propriété de piégeage des radicaux libres peut trouver une autre application dans la lutte contre le stress oxydatif et donc dans le traitement ou la prévention de ses conséquences comme de nombreuses maladies par exemple la cataracte, l'arthrite, les maladies cardio-vasculaires ou les cancers.

On sait par ailleurs que la mélanine peut neutraliser les effets nocifs des rayonnements qu'ils soient ionisants ou non.

Ainsi, la mélanine pourrait absorber une grande quantité d'énergie et ne produirait que peu de chaleur lorsqu'elle absorbe cette énergie. Ainsi il a été avancé que la mélanine pourrait absorber de grosses quantités d'énergie de toutes sortes, y compris l'énergie des rayons du soleil, des machines à rayons X, et l'énergie formée dans les cellules pendant le métabolisme de celles-ci.

Ainsi l'invention a encore pour objet le complexe protéomélanique bioassimilable selon l'invention pour protéger les organismes vivants des rayonnements ionisants (Rayonnement ultraviolet (lointain), Rayon X, Rayon gamma, Neutron, Électron / particule β⁻, Positron / particule β⁺, Muon, Proton, Ion ⁴He / particule a, Ion ¹²C) ou non (Ultraviolet (proche) A, B et/ou C, Lumière visible, Infrarouge A, B et/ou C, Microondes, Ondes radio).

Préférentiellement, l'invention a pour objet le complexe protéomélanique bioassimilable selon l'invention pour protéger les organismes vivants du rayonnement ultraviolet, lointain ou proche (A, B ou C), de la lumière visible, du rayonnement Infrarouge (A, B et/ou C) ou encore des Rayons X.

Par ailleurs, la mélanine est connue pour fixer et concentrer les métaux lourds dans les plumes chez les oiseaux. Il est donc envisageable d'utiliser le complexe protéomélanique bioassimilable selon l'invention pour piéger les métaux lourds, avantageusement ceux de l'organisme, avantageusement l'organisme humain, et ainsi détoxifier ledit organisme.

L'invention a encore pour objet l'utilisation du complexe protéomélanique bioassimilable selon l'invention dans la préparation de compositions cosmétique, dermatologique, pharmaceutique ou encore alimentaire.

L'invention a également pour objet une composition comprenant au moins un complexe protéomélanique bioassimilable selon l'invention. Ladite composition peut être une composition cosmétique, dermatologique, pharmaceutique ou encore alimentaire, en conséquence de quoi outre le complexe protéomélanique bioassimilable ladite composition peut comprendre tout autre composé et/ou adjuvant habituellement utilisé dans les domaines de la cosmétique, de la dermatologie, de la pharmacie ou de l'alimentaire.

Une composition particulièrement préférée selon l'invention peut comprendre du complexe protéomélanique bioassimilable selon l'invention, de la tyrosine et/ou de la cystéine, avantageusement sous forme libre ou combinée ou sous forme de peptide. Cette composition est particulièrement adaptée comme complément alimentaire.

Selon l'invention ladite composition peut comprendre du complexe protéomélanique bioassimilable en une quantité comprise entre 1% et 100 %, préférentiellement comprise entre 20% et 95 %, très préférentiellement comprise entre 50% et 95 %, en poids par rapport au total de la composition.

Cette composition est particulièrement adaptée pour favoriser la mélanogénèse.

Ladite composition pourra prendre toutes les formes galénique connues et compatibles avec son usage, particulièrement la forme d'une crème, d'une solution, d'un onguent, d'une lotion, d'un savon, d'une mousse, d'un shampoing, d'un déodorant, d'un démaquillant, d'un mascara, d'un eyeliner, d'un rouge à lèvre, d'un gel, de préparation pressurisée (spray), ces formes étant plus particulièrement adaptées pour une utilisation en application sur la peau et/ou les cheveux ou encore la forme d'un comprimé, d'un sirop, d'une poudre ingérable, d'une gélule, d'une capsule molle, d'une capsule dure, d'une gomme à mâcher, de granulés, d'un pré-mélange pour aliment, d'une préparation liquide, d'une préparation semi solide, ces formes étant plus particulièrement adaptées pour une utilisation par ingestion par voie orale. D'autres formes d'usage plus spécifique peuvent être envisagées comme une préparation vaginale, une préparation rectale, une préparation oculaire, des tampons médicamenteux ou un pansement imprégné.

Bien évidement ces compositions peuvent en outre comprendre tout autre composé ou adjuvant adaptés à la forme galénique choisie. A cet égard on peut citer les antimousses, les catalyseurs, les agents de clarification/adjuvants de filtration, les agents décolorants, les agents de lavage et de pelage/épluchage, les agents de plumaison et de pelage/épilation, les résines échangeuse d'ions, les agents de congélation par contact et agents de refroidissement, les agents de dessiccations/antiagglomérants, les enzymes, les agents d'acidification, d'alcalinisation ou de neutralisation, les agents de démoulages, les floculants et coagulants, les agents de décontamination, les antitartres, les solvants d'extraction, les huiles minérales, naturelles, d'extractions, les substances chimiquement réactives, oxydantes, les vitamines, les sels minéraux, les excipients, les tensioactifs, les conservateurs, les filtres ultraviolets, les colorants, les antioxydants, les alginates, les édulcorants, les exhausteurs de goût, les cires et hydrocarbures, les gommes à mâcher, les gaz propulseurs, les inerteurs, les conditionneurs ou traceurs.

L'invention a encore pour objet l'utilisation d'une composition selon l'invention, pour introduire dans l'organisme, par voie non chirurgicale, de la mélanine exogène, particulièrement de la mélanine exogène ayant conservé ses propriétés intrinsèques, ou pour stimuler la mélanogénèse, particulièrement dans la peau, dans les yeux ou dans les phanères, préférentiellement dans les cheveux, les poils, la laine, ou pour protéger les organismes vivants du rayonnement ultraviolet, ou pour piéger les radicaux libres, avantageusement combattre leurs effets néfastes comme le vieillissement tissulaire, en particulier l'apparition des rides et des ridules de la peau, ou pour lutter contre le stress oxydatif et traiter ou prévenir ses conséquences comme par exemple la cataracte, l'arthrite, les maladies cardio-vasculaires ou les cancers ou pour neutraliser les effets potentiellement nocifs de rayonnements autres que le rayonnement UV ou pour piéger les métaux lourds, avantageusement ceux de l'organisme et ainsi détoxifier l'organisme ou encore pour la préparation d'une composition pharmaceutique destinée à la prévention et/ou au traitement de la maculopathie (ARM) et/ou de la dégénération maculaire (DMLA).
Figure 1 : Schéma illustrant les zones d'expérimentation utilisées au cours des essais.
Figure 2 : Illustration de la variation des paramètres L* (2A) et ITA° (2B) dans l'étude de l'effet du complément alimentaire comprenant le complexe protéomélanique bioassimilable (produit), sur la pigmentation cutanée immédiate (exposition UVA).
Figure 3 : Illustration de la variation de l'index mélanique (IM) dans l'étude de l'effet du complément alimentaire comprenant le complexe protéomélanique bioassimilable (produit), sur la pigmentation cutanée immédiate (exposition UVA).
Figure 4 : Illustration de la variation des paramètres L* (4A) et ITA° (4B) dans l'étude de l'effet du complément alimentaire comprenant le complexe protéomélanique bioassimilable (produit), sur la pigmentation retardée (exposition UVB/UVA).
Figure 5 : Illustration de la variation de l'index mélanique (IM) dans l'étude de l'effet du complément alimentaire comprenant le complexe protéomélanique bioassimilable (produit), sur la pigmentation retardée (exposition UVB/UVA).
Figure 6 : Illustration de la variation des paramètres L* (6A) et ITA° (6B) dans l'étude de l'effet du complément alimentaire comprenant le complexe protéomélanique bioassimilable (produit), sur une zone non exposée.
Figure 7 : Illustration de la variation de l'index mélanique (IM) dans l'étude de de l'effet du complément alimentaire comprenant le complexe protéomélanique bioassimilable (produit), sur une zone non exposée.
Figure 8 : Illustration de la variation des paramètres L* dans l'étude de l'effet du complément alimentaire comprenant le complexe protéomélanique bioassimilable (produit), sur la coloration des cheveux.
Figure 9 : Illustration des résultats de l'étude de bioassimilabilité des éléments du complexe protéomélanique selon l'invention.
Figure 10 : Courbe étalon établie pour déterminer la quantité de mélanine présente dans les échantillons obtenus en fin de l'étude de bioassimilabilité.
Figure 11 : Illustration des résultats de l'étude de la modification de la teneur en protéines de la peau pour les complexes protéomélanique. [□Témoin, ■Groupe Actif].

D'autres éléments, caractéristiques et avantages de l'invention pourront apparaitre à la lecture des exemples suivants donnés à titre illustratif ainsi que de l'observation des figures annexées.

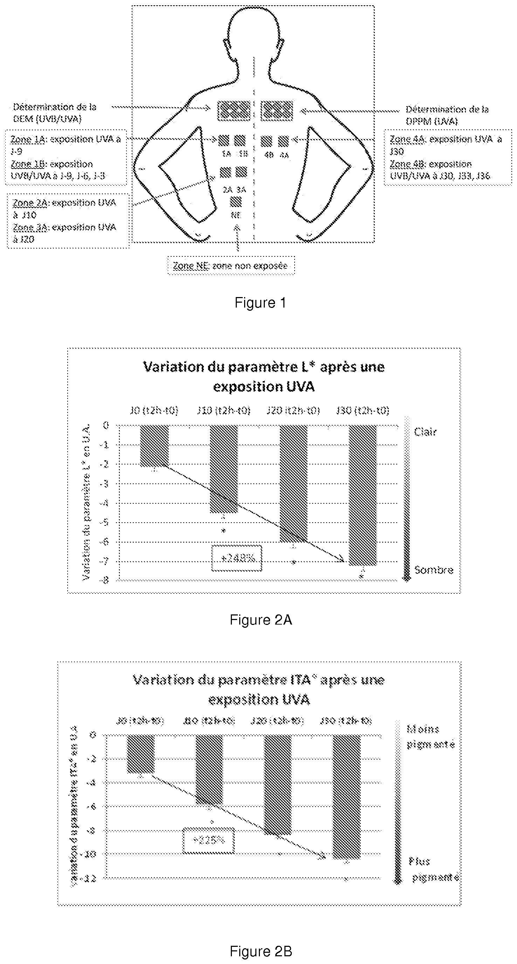

La figure 1, illustre l'emplacement des zones de peaux soumises aux irradiations UV au cours des essais présentés en exemples. On note la présence d'une zone de mesure de la Dose Erythémale Minimale (DEM) définie comme la plus petite dose de rayonnement UltraViolet (UV) induisant le premier érythème perceptible apparaissant sur la majeure partie du site d'exposition aux UV, dans les 16 à 24 heures après l'exposition, et d'une zone de mesure de la dose minimale de pigment persistant (DPPM) définie comme la Dose Pigmentaire Persistante Minimale (DPPM).

Les différentes zones témoins 1A et 1B reçoivent des doses d'UV définies (DEM et DPPM) et permettent de mesurer la réponse épidermique avant la prise du produit protéomélanique.

Les zones 2A, 3A et 4A permettent de mesurer la réponse épidermique après la prise du produit protéomélanique sous UVA tous les 10 jours. Cela correspond à la mesure de la pigmentation immédiate due à la mélanine qui migre à la surface de la peau en réponse au rayonnement UVA.

La Zone 4B permet de mesurer la réponse épidermique pendant la prise du produit protéomélanique et sous UV A/B. Cela correspond à la mesure de la pigmentation retardée due à la production accrue de mélanine mesurée après 30 jours de cure et qui se traduit par un bronzage plus intense et persistant en réponse, ce qui correspond à l'activation de la synthèse de la mélanine (mélanogénèse).

La Zone NE et la zone de mesure avant et après la prise du complexe, sans irradiation UV. Cela correspond à la recherche de l'activité du produit sur la mélanogénèse hors irradiation.

La figure 2, illustre les résultats obtenus dans l'étude de la variation des paramètres L* (2A) et ITA° (2B) après administration du complément alimentaire comprenant le complexe protéomélanique bioassimilable (produit), sur la pigmentation cutanée immédiate (exposition UVA). On note qu'à J0 deux heures après une exposition aux UVA, la peau était significativement plus sombre (diminution du paramètre L* ; figure 2A) et plus pigmentée (diminution du paramètre ITA° ; Figure 2B) après l'utilisation du complément alimentaire comprenant le complexe protéomélanique bioassimilable (produit) selon l'invention.

Cet effet a été observé dès 10 jours d'utilisation du produit et l'efficacité augmente encore avec le temps (effet maximum observé après 30 jours d'utilisation du produit : diminution de 225% du paramètre ITA° comparé à avant utilisation du produit.). Ces résultats sont statistiquement significatifs (p<0,001%).

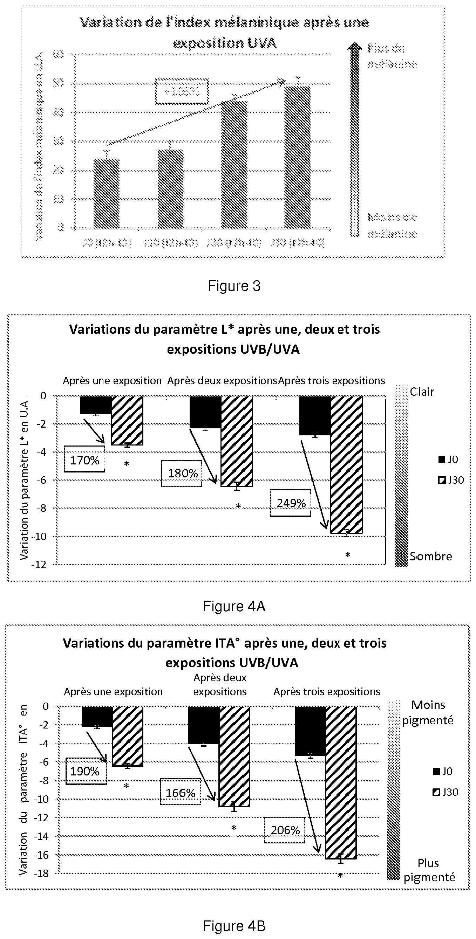

La figure 3 illustre les résultats obtenus dans l'étude de la variation de l'index mélanique (IM) après administration du complément alimentaire comprenant le complexe protéomélanique bioassimilable (produit), sur la pigmentation cutanée immédiate (exposition UVA). On note qu'après 10 jours d'utilisation du produit, les mesures au Mexamètre® ont montré une peau légèrement plus pigmentée, deux heures après l'exposition UVA (+14%, variation à la limite de la significativité). Après 20 et 30 jours d'utilisation du produit, l'augmentation de l'index mélanique est beaucoup plus important et devient statistiquement significatif (à J30 : +106%, p<0,001).

La figure 4 illustre les résultats obtenus dans l'étude de la variation des paramètres L* (4A) et ITA° (4B) après administration du complément alimentaire comprenant le complexe protéomélanique bioassimilable (produit), sur la pigmentation retardée (exposition UVB/UVA). On note qu'après 30 jours d'utilisation du complément alimentaire comprenant le complexe protéomélanique bioassimilable (produit), les mesures au Spectrocolorimètre® ont montré une peau significativement plus sombre (diminution du paramètre L*) et plus pigmentée (diminution du paramètre ITA°) après une, deux ou trois expositions UVB/UVA (après trois expositions : diminution significative du paramètre ITA° de 206% comparé à avant l'utilisation du produit).

Ces résultats sont statistiquement significatifs (p<0,001 %).

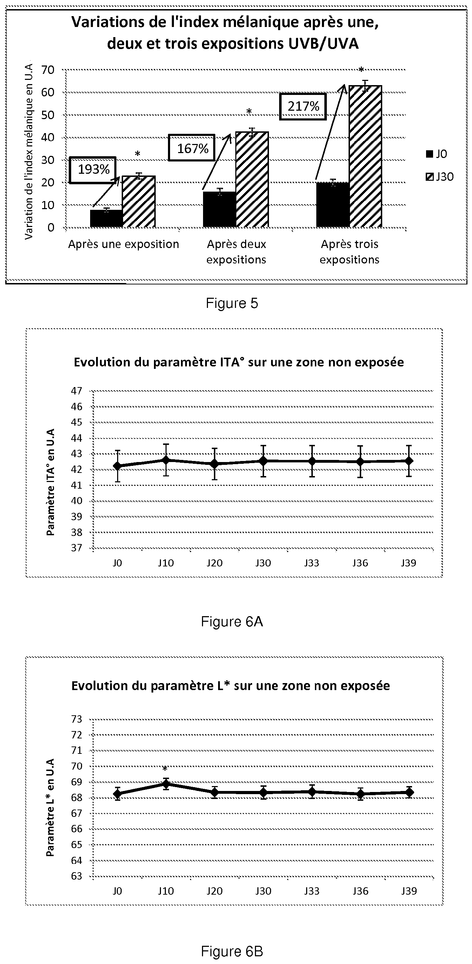

La figure 5 illustre les résultats obtenus dans l'étude de la variation de l'index mélanique (IM) après administration du complément alimentaire comprenant le complexe protéomélanique bioassimilable (produit), sur la pigmentation retardée (exposition UVB/UVA). On note qu'après 30 jours d'utilisation du complément alimentaire comprenant le complexe protéomélanique bioassimilable (produit), les mesures au Mexamètre® ont montré une peau significativement plus pigmentée (augmentation de l'index mélanique) après une, deux ou trois expositions UVB/UVA (après trois expositions, augmentation de l'index mélanique de 217% comparé à avant utilisation). Ces résultats sont statistiquement significatifs (p<0,001%).

La figure 6 illustre les résultats obtenus dans l'étude de la variation des paramètres L* (6A) et ITA° (6B) après administration du complément alimentaire comprenant le complexe protéomélanique bioassimilable (produit), sur une zone non exposée. On note que durant toute la prise du complément alimentaire comprenant le complexe protéomélanique bioassimilable (produit), les mesures au Spectrocolorimètre® n'ont pas montré de variation pertinente de la couleur de la peau sur une zone non exposée aux UV : variations de 0% à 2%, non significatives pour la plupart et non pertinentes du point de vue biologique (non visible à l'oeil nu).

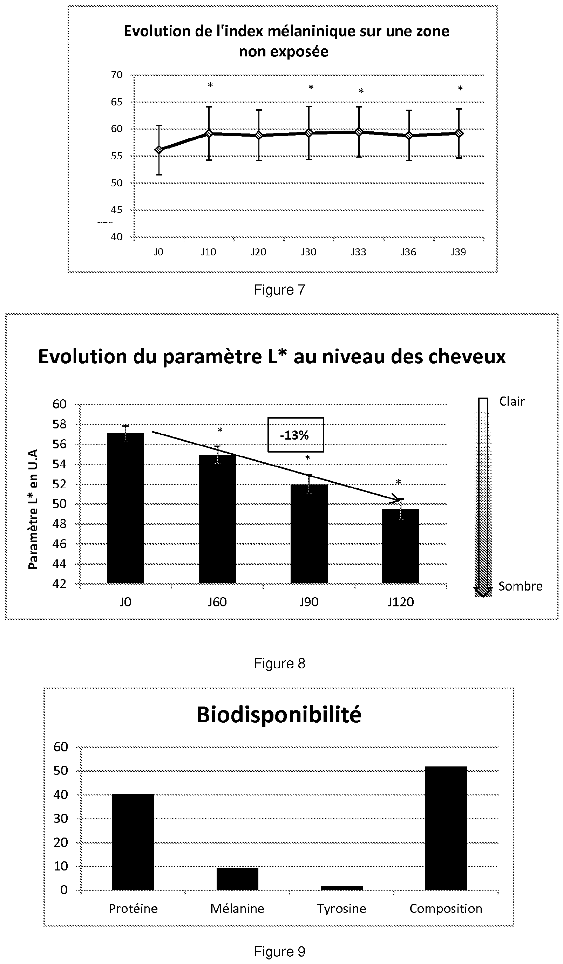

La figure 7 illustre les résultats obtenus dans l'étude de la variation de l'index mélanique (IM) après administration du complément alimentaire comprenant le complexe protéomélanique bioassimilable (produit), sur une zone non exposée. On note qu'entre 10 et 39 jours d'utilisation du complément alimentaire comprenant le complexe protéomélanique bioassimilable (produit), les mesures au Mexamètre® ont montré une très légère augmentation de l'index mélanique (variation significative ou à la limite de la significativité entre 4% et 6%).

La figure 8 illustre les résultats obtenus dans l'étude de la variation des paramètres L* dans l'étude de l'effet du complément alimentaire comprenant le complexe protéomélanique bioassimilable (produit), sur la coloration des cheveux. On note que pendant les 120 jours d'utilisation du complément alimentaire comprenant le complexe protéomélanique bioassimilable (produit), les mesures au Spectrocolorimétre® ont montré une couleur des cheveux significativement plus sombre (13% après 120 jours d'utilisation, p<0,001). L'effet augmente avec le temps de traitement. Une variation de la teinte des cheveux a également été observée avec une couleur moins "jaune" et moins "rouge".

La figure 9 illustre les résultats de l'étude de bioassimilabilité du complexe protéomélanique selon l'invention. On note une bioassimilabilité des protéines dans les liquides gastro-intestinaux simulés de 40,5 ± 0,8%, une bioassimilabilité de la tyrosine égale à 1,85 ± 0,2%, et une bioassimilabilité de la mélanine dans les fluides gastro-intestinaux simulés de 9,43 ± 0,7%.

La bioassimilabilité totale du complexe protéomélanique a été mathématiquement déterminée en ajoutant la teneur en protéines, en tyrosine et en mélanine; il en résulte une valeur de la bioassimilabilité du complexe protéomélanique de 49,93%(figure 9).

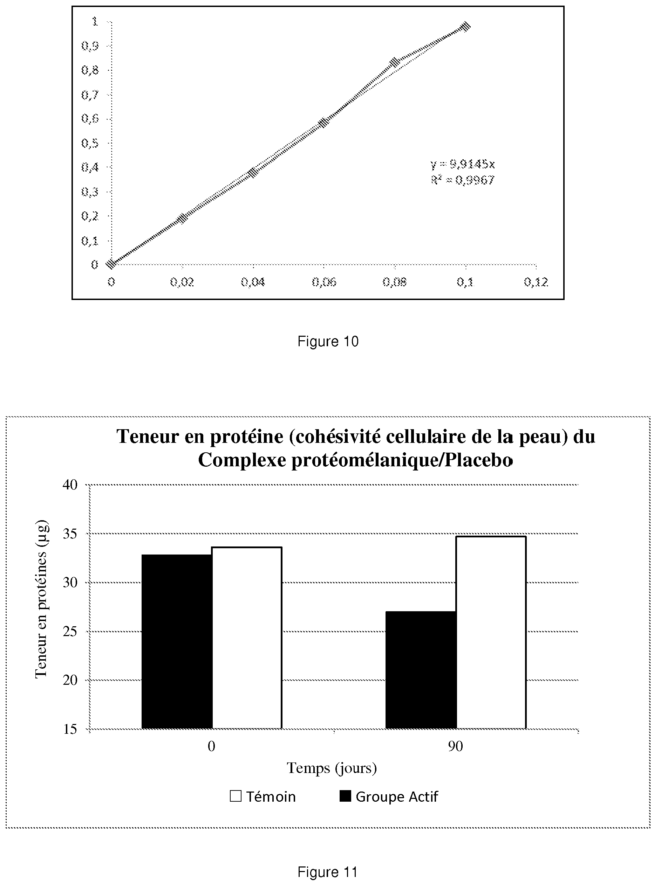

La figure 10 montre la courbe utilisée pour la détermination quantitative de mélanine par méthodes spectrophotométriques (voir exemple 5).

La figure 11 illustre les résultats de la modification de la teneur en protéines de la peau recueillies chez les sujets ayant reçu le complexe protéomélanique selon l'invention [Groupe Actif] au regard d'un groupe de sujet n'ayant pas reçu ledit complexe protéomélanique [Témoin].

La teneur en protéines (cohésivité) est mesurée en utilisant la méthode Corneofix _ feuille (Courage + Khazaka, electronic GmbH).

Les feuilles du Corneofix® F 20 sont déposées sur les couches cornées d'un visage propre à T0 et T90 jours. La détermination de la teneur en protéines captées dans les feuilles du Corneofix® F 20 permet l'évaluation de la cohésivité de la peau.

Une amélioration de la cohésivité de la peau implique une diminution de la quantité de protéines captées par la feuille entre les prélèvements à T0 et à T90 jours.

La prise de complexe protéomélanique a significativement amélioré la cohésion de la peau par rapport au témoin au jour 90 avec une diminution de 19,7% de la teneur en protéine par rapport au témoin (P <0,001).

### Exemple 1 : Préparation d'un complexe protéomélanique bioassimilable selon l'invention

### Première étape : sulfitolyse oxydative appliquée à la laine

### Mode opératoire :

100 kg de laine de mouton de race "Noire du Velay" dégraissée lavée sont introduits dans une cuve.

### Préparation de la solution cuivre-ammonium et de la solution sulfites :

### Préparation du complexe cuivre ammoniaque soluble en milieu basique :

Dans une autre cuve, on mélange environ 20 litres d'une solution aqueuse d'ammoniaque à 25% avec 8 kg de cuivre pur, sous forme de sulfate de cuivre. On ajoute alors environ 750 litres d'eau dans lesquels on aura au préalable dissous 50 kg de sulfites sodiques.

### Déroulement de la réaction, paramètres contrôlés :

Le mélange réactionnel est ajouté dans la cuve contenant la laine puis mis en mouvement. La température est maintenue entre 20 et 40 °C, et de l'air est injecté pour apporter de l'oxygène. Lorsque le milieu réactionnel est devenu suffisamment fluide le mélange est agité. Cette opération est conduite pendant une durée de 1 à 2 jours environ, jusqu'à ce que les fibres aient perdu toute cohésion entre elles.

### Deuxième étape : hydrolyse et inactivation enzymatique

On ajoute alors 350 g d'enzyme Protex 6L de chez Genencor ou Multifect PR 6L de chez Brenntag (alcaline sérine protéase) dont l'activité enzymatique est 580 000 U/g dans le milieu à un pH maintenue-entre 9 et 10, et à une température comprise entre 40 et 50°C;
On laisse hydrolyser pendant 24 heures sous agitation.

Au fur et à mesure de l'hydrolyse, le pH augmentant, de la soude est ajoutée afin de maintenir constant le pH entre 9 et 10

Lorsque le pH ne varie plus, donc qu'il n'est plus nécessaire d'ajouter de la soude, ou lorsqu'on estime que l'hydrolyse est suffisante, l'étape d'hydrolyse est alors terminée.

Le milieu réactionnel contient alors entre autre le complexe protéomélanique objet de l'invention qui peut être utilisé en l'état ou purifié et concentré par toutes techniques connues par l'homme du métier.

### Exemple 2 : Composition comprenant du complexe protéomélanique obtenu à l'exemple 1.

2A) lotion pour ralentir la chute et recolorer les cheveux blancs par application topique sur cuir chevelu, cheveu

| | |
|---|---|
| Complexe protéomélanique | 7,0 g |
| Urée | 1,0 g |
| Lactate de sodium | 0,5 g |
| Phénoxyéthanol | 0,1 g |
| Parfum | qs |
| Eau qsp | 100,0 g |

2B) crème préparatrice, accélératrice du bronzage et régénératrice de l'épiderme

| | |
|---|---|
| Complexe protéomélanique | 5,0 g |
| Behenate de glycérol | 2,0 g |
| Isononyl isononanoate | 3,0 g |
| Beurre de karité | 1,5 g |
| Glycérine | 4,0 g |
| Tocophérol | 0,1 g |
| Huile de vaseline | 8,0 g |
| Ester de polyglycérol | 0,5 g |
| Parfum | qs |
| Undecylanate de glycérol | 0,1 g |
| Eau | qsp 100,0 g |

2C) Complément alimentaire préparateur et accélérateur de bronzage, sous forme de capsule

| | |
|---|---|
| Complexe protéomélanique sous forme poudre microgranulée | 325,0 mg |
| Vitamine C | 90,0 mg |
| Gluconate de cuivre | 1,0 mg |
| Stéarate de magnésium | 2,0 mg |
| Oxyde de silice | 0,2 mg |
| 1 Capsule dure en gélatine | 94,0 mg |

### Exemple 3 : Evaluation de l'effet d'un complexe protéomélanique bioassimilable selon l'invention sur la pigmentation de la peau.

L'objectif de cette étude est d'évaluer l'effet du complexe protéomélanique bioassimilable selon l'invention, préparé à l'exemple 1, sur la pigmentation de la peau sous stimulation UV, après 10, 20 et 30 jours d'utilisation du produit étudié.

Au cours de cette évaluation ont été étudiés :
- l'effet du produit sur la pigmentation de la peau après exposition aux UVA et mesure de la pigmentation immédiate (2 heures après exposition) avec un Spectrocolorimètre® et un Mexamètre® après 10, 20 and 30 jours d'utilisation du produit. Cette pigmentation implique principalement l'activation de la mélanine déjà présente dans la peau.
- l'effet du produit sur la pigmentation de la peau après exposition répétées aux UV(B+A) et mesure de la pigmentation retardée avec un Spectrocolorimètre® et un Mexamètre® après 30 jours d'utilisation du produit. Cette pigmentation implique principalement la synthèse de nouvelle mélanine.

### 3.1) Outils et protocoles

### Expositions UV

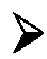 Pour mesurer la Dose Erythémale Minimale (DEM : plus petite dose de rayonnement UltraViolet (UV) induisant le premier érythème perceptible apparaissant sur la majeure partie du site d'exposition aux UV, dans les 16 à 24 heures après l'exposition et de la Dose Pigmentaire Persistante Minimale (DPPM) une Lampe Xenon de type Solar Light Multiport 601-300W, munie d'un filtre WG320 (1,25 mm) pour les UV(A+B) et d'un filtre WG335 (3 mm)(UVA) pour éliminer les UVB et d'une puissance de300 W a été utilisée.

Les spectres émis sont pour les UV (A+B) de 290 à 400 nm et pour les UVA de 320 à 400 nm.

Un filtre UG11 (1mm) et miroir dichroïque ont été utilisés pour éliminer les rayonnements Infra-rouges et la lumière visible.

La surface de peau exposée est délimitée à l'aide d'un cache comportant six trous (diamètre 8 mm) ≥ 0,5cm².

Pour déterminer la DEM le flux d'UV de chaque fibre optique est réglé par le technicien pour obtenir une progression géométrique de 15%. Le système est utilisé avec un flux constant, toutes les fibres sont ouvertes en même temps.

Pour déterminer la DPPM le flux d'UVA de chaque fibre optique est déterminé par le technicien pour obtenir une progression géométrique de 25%. Le système est utilisé avec un flux constant, toutes les fibres sont ouvertes en même temps.

Pour les expositions aux UV (A+B) et UVA, une lampe Xenon de type Solar Light Monoport 1000W High Power Solar Simulator - Model LS1000, présentant un spectre UVA (A+B) de 290 à 400 nm et UVA de 320 à 400 nm a été utilisée. La surface de peau exposée est au maximum 4 x 4 cm.

### Mesures au Spectrocolorimètre® au niveau de la peau

La mesure colorimétrique de la peau est effectuée à l'aide d'un Spectrocolorimètre® MINOLTA CM700-d, muni d'une tête de 8 mm de diamètre.

Le Spectrocolorimètre® convertit les couleurs situées dans la plage de perception humaine en un code numérique composé de trois paramètres :
**L*** : représente la clarté (du sombre au pâle),
**a*** : représente la gamme des verts aux rouges,
**b*** : représente la gamme des bleus aux jaunes.
a* et b* sont des paramètres de chrominance et L* un paramètre de luminance.

Il devient alors possible d'exprimer, dans les moindres détails, les différences entre deux zones cutanées qui paraissent être de la même couleur. Après calibration, les mesures sont réalisées directement sur la peau à l'aide d'une source de lumière Xénon pulsée et d'un système à double faisceaux pour mesurer la lumière émise et corriger toute légère déviation.

Cet instrument est couramment utilisé en cosmétique et en médecine pour mesurer la couleur de la peau.

Les paramètres L* (caractéristique de la clarté) et b* (caractéristique de la pigmentation jaune mélanique cutanée) sont étudiés lors d'une étude sur la pigmentation cutanée.

Ces deux paramètres sont exploités à travers le calcul de "l'Angle Typologique Individuel" (ITA), qui définit le degré de pigmentation de la peau d'une personne en intégrant la clarté (L*) et le paramètre de mélanisation (b*), selon la formule suivante :
ITA° = [Arc tan((L*-50)/b*)] x 180 / π
Plus l'ITA° est élevé, plus la peau est claire.
Chaque mesure est la moyenne de trois acquisitions.

### Mesures au Mexamètre® au niveau de la peau

Le Mexamètre® est un instrument de Courage et Khazaka, muni d'une tête de 5 mm de diamètre, qui mesure particulièrement le contenu en mélanine et en hémoglobine de la peau. Ces deux composants sont principalement responsables de la couleur de la peau.

La mesure est basée sur le principe d'absorption. La sonde spéciale du Mexamètre® MX18 émet de la lumière à trois longueurs d'onde prédéfinies (568nm (vert), 660nm (rouge) et 880nm (infra-rouge)). Un photo-détecteur mesure la lumière réfléchie par la peau. Cette mesure est basée sur le même principe optique qui consiste en la mesure de la lumière absorbée et réfléchie aux longueurs d'onde du rouge et de l'infra-rouge pour la mélanine.

Un Index de Mélanine (Mx) est calculé à partir de l'intensité de la lumière absorbée et réfléchie à respectivement 600 et 880 nm.

Un Index d'Erythème (Ex) est calculé à partir de l'intensité de la lumière absorbée et réfléchie à respectivement 568 et 660 nm.

Seul l'Index de Mélanine, qui représente le contenu en mélanine de la peau a été analysé. Une augmentation de ce paramètre caractérise une augmentation de la pigmentation de la peau.

Chaque mesure est la moyenne de trois acquisitions.

### Déroulement de l'essai

L'étude a été réalisée sur 35 sujets

### A J-10

Les sujets viennent au laboratoire sans avoir appliqué un quelconque produit sur le dos depuis la veille au soir et les zones d'exposition au niveau du dos sont définies comme présenté à la figure 1
- Exposition UVB/UVA pour la détermination de la DEM.
- Exposition UVA pour la détermination de la DPPM.
- 2 heures après exposition UVA : lecture de la DPPM

### AJ-9

- Les sujets viennent au laboratoire sans avoir appliqué de produit sur le dos depuis la veille au soir.
- Entre 16 et 24 heures après exposition UVB/UVA : lecture de la DEM.
- Mesures au Spectrocolorimètre® sur les zones 1A, 1B et NE.
- Mesures au Mexamètre® sur les zones 1A, 1B et NE.
- Exposition UVB/UBA sur la zone 1B à 0,8 DEM.
- Exposition UVA sur la zone 1A à 1,25 DPPM.
- 2 heures après exposition UVA : mesures au Spectrocolorimètre® et Mexamètre® sur la zone 1A.

### A J-6

- Les sujets viennent au laboratoire sans avoir appliqué de produit sur le dos depuis la veille au soir.
- Mesures au Spectrocolorimètre® et Mexamètre® sur les zones 1B et NE.
- Exposition UVB/UBA sur la zone 1B à 0,8 DEM.

### A J-3

- Les sujets viennent au laboratoire sans avoir appliqué de produit sur le dos depuis la veille au soir.
- Mesures au Spectrocolorimètre® et Mexamètre® sur les zones 1B et NE.
- Exposition UVB/UBA sur la zone 1B à 0,8 DEM.

### A J0

- Les sujets viennent au laboratoire sans avoir appliqué de produit sur le dos depuis la veille au soir.
- Mesures au Spectrocolorimètre® et Mexamètre® sur les zones 1B et NE.

Le produit est pris à raison de 2 capsules par jour le matin pendant le petit-déjeuner et ce jusqu'à la fin de l'étude.

### A J10

- Les sujets viennent au laboratoire sans avoir appliqué de produit sur le dos depuis la veille au soir.
- Mesures au Spectrocolorimètre® et Mexamètre® sur les zones 2A et NE.
- Exposition UVA sur la zone 2A à 1,25 DPPM.
- 2heures après exposition UVA : mesures au Spectrocolorimètre® et Mexamètre® sur la zone 2A.

### A J20

- Les sujets viennent au laboratoire sans avoir appliqué de produit sur le dos depuis la veille au soir.
- Mesures au Spectrocolorimètre® et Mexamètre® sur les zones 3A et NE.
- Exposition UVA sur la zone 3A à 1,25 DPPM.
- 2heures après exposition UVA : mesures au Spectrocolorimètre® et Mexamètre® sur la zone 3A.

### A J30

- Les sujets viennent au laboratoire sans avoir appliqué de produit sur le dos depuis la veille au soir.
- Mesures au Spectrocolorimètre® sur les zones 4A, 4B et NE.
- Mesures au Mexamètre® sur les zones 4A, 4B et NE.
- Exposition UVB/UBA sur la zone 4B à 0,8 DEM.
- Exposition UVA sur la zone 4A à 1,25 DPPM.
- 2heures après exposition UVA : mesures au Spectrocolorimètre® et Mexamètre® sur la zone 4A.

### A J33

- Les sujets viennent au laboratoire sans avoir appliqué de produit sur le dos depuis la veille au soir.
- Mesures au Spectrocolorimètre® et Mexamètre® sur les zones 4B et NE.
- Exposition UVB/UBA sur la zone 4B à 0,8 DEM.

### A J36

- Les sujets viennent au laboratoire sans avoir appliqué de produit sur le dos depuis la veille au soir.
- Mesures au Spectrocolorimètre® et Mexamètre® sur les zones 4B et NE.
- Exposition UVB/UBA sur la zone 4B à 0,8 DEM.

### A J39

- Les sujets viennent au laboratoire sans avoir appliqué de produit sur le dos depuis la veille au soir.
- Mesures au Spectrocolorimètre® et Mexamètre® sur les zones 4B et NE.

### Analyse des données

L'analyse statistique des données permet de déterminer la significativité des variations sous l'effet du produit testé.

La comparaison porte sur les valeurs obtenues aux différents temps d'évaluation.

Le test utilisé est le test t de Student sur données appariées. Les conditions d'application sont le caractère aléatoire et simple des échantillons et la normalité de la population des différences.

Le principe du test consiste à poser une hypothèse nulle (H0) d'absence de différence entre l'effet moyen aux différents temps d'évaluation (*d̅* = 0) et une hypothèse alternative H1 (notre hypothèse de recherche) d'une différence entre les temps d'évaluation (*d̅* <> 0).

On détermine ensuite quelle est la probabilité p d'observer un écart entre les temps au moins aussi grand que celui qui a été observé si l'hypothèse nulle est vraie.
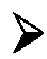 Si p ≤ 5%, on rejette l'hypothèse nulle. On accepte alors l'hypothèse alternative H1 d'une différence significative entre les temps d'évaluation.
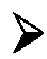 Si p > 5%, on accepte l'hypothèse nulle. Les données n'ont pas permis de mettre en évidence une différence significative entre les temps d'évaluation.

### 3.2) Résultats

Dans les tableaux présentés ci-après les résultats de L* sont donnés de sombre à clair, les résultats de ITA° sont donnés de plus pigmenté à moins pigmenté.

### 3.2.1) Effet du complément alimentaire comprenant le complexe protéomélanique bioassimilable (produit), sur la pigmentation cutanée immédiate (exposition UVA) Spectrocolorimètre®

Une synthèse des résultats est présentée dans le tableau 1 ci-dessous.

**Tableau 1**

| | J10 | | | J20 | | | J30 | | |
|---|---|---|---|---|---|---|---|---|---|
| | Variation | | p | Variation | | p | Variation | | p |
| | UA | % | | UA | % | | UA | % | |
| L* | -2,4 +/- 0,3 | 118 | <0,001 | -3,9 +/- 0,4 | 189 | <0,001 | -6,1 +/- 0,4 | 248 | <0,001 |
| ITA° | -2,6 +/- 0,4 | 82 | <0,001 | -6,2 +/- 0,4 | 161 | <0,001 | -7,2 +/- 0,5 | 225 | <0,001 |

| | | | | | | | | | |
|---|---|---|---|---|---|---|---|---|---|
| UA : moyenne +/- SEM | | | | | | | | | |

Ces résultats sont également illustrés dans les figures 2A et 2B.

Deux heures après une exposition UVA, la peau était significativement plus sombre (diminution du paramètre L*) et plus pigmentée (diminution du paramètre ITA°) après l'utilisation du complément alimentaire comprenant le complexe protéomélanique bioassimilable (produit) selon l'invention.

Cet effet a été observé dès 10 jours d'utilisation du produit et l'efficacité augmente encore avec le temps (effet maximum observé après 30 jours d'utilisation du produit : diminution de 225% du paramètre ITA° comparé à avant utilisation du produit.)

### Mexamètre®

Une synthèse des résultats est présentée dans le tableau 2 ci-dessous.

**Tableau 2**

| | J10 | | | J20 | | | J30 | | |
|---|---|---|---|---|---|---|---|---|---|
| | Variation | | p | Variation | | p | Variation | | p |
| | UA | % | | UA | % | | UA | % | |
| IM | 3,3 +/- 1,8 | 14 | 0,076 | 20 +/- 1,9 | 84 | <0,001 | 25,3 +/- 3,6 | 106 | <0,001 |

| | | | | | | | | | |
|---|---|---|---|---|---|---|---|---|---|
| IM = Index Mélanique ; UA : moyenne +/- SEM | | | | | | | | | |

Ces résultats sont également illustrés dans la figure 3

Après 10 jours d'utilisation du produit, les mesures au Mexamètre® ont montré une peau légèrement plus pigmentée, deux heures après l'exposition UVA (+14%, variation à la limite de la significativité). Après 20 et 30 jours d'utilisation du produit, l'augmentation de l'index mélanique est beaucoup plus important et devient statistiquement significatif (à J30 : +106%, p<0,001).

### 3.2.2) Effet du complément alimentaire comprenant le complexe protéomélanique bioassimilable (produit), sur la pigmentation retardée (exposition UVB/UVA) Spectrocolorimètre®

Une synthèse des résultats est présentée dans le tableau 3 ci-dessous.

**Tableau 3**

| | | J30 | | |
|---|---|---|---|---|
| | | Variations | | p |
| | | UA | % | |
| Après 1 exposition aux UV | L* | -2,2 +/- 0,0 | 170 | <0,001 |
| | ITA° | -4,2 +/- 0,3 | 190 | <0,001 |
| Après 2 expositions aux UV | L* | -4,1 +- 0,3 | 180 | <0,001 |
| | ITA° | -6,7 +/- 0,6 | 166 | <0,001 |
| Après 3 expositions aux UV | L* | -7,0 +/- 0,3 | 249 | <0,001 |
| | ITA° | -11,0 +/- 0,5 | 206 | <0,001 |

Ces résultats sont également illustrés dans les figures 4A et 4B

Après 30 jours d'utilisation du complément alimentaire comprenant le complexe protéomélanique bioassimilable (produit), les mesures au Spectrocolorimètre® ont montré une peau significativement plus sombre (diminution du paramètre L*) et plus pigmentée (diminution du paramètre ITA°) après une, deux ou trois expositions UVB/UVA (après trois expositions : diminution significative du paramètre ITA° de 206% comparé à avant l'utilisation du produit).

### Mexamètre®

Une synthèse des résultats est présentée dans le tableau 4 ci-dessous.

**Tableau 4**

| | | J30 | | |
|---|---|---|---|---|
| | | Variations | | p |
| | | UA | % | |
| IM | Après 1 exposition aux UV | 15,1 +/- 0,0 | 193 | <0,001 |
| | Après 2 expositions aux UV | 26,5 +- 1,9 | 167 | <0,001 |
| | Après 3 expositions aux UV | 43,1 +/- 2,7 | 217 | <0,001 |

| | | | | |
|---|---|---|---|---|
| IM = Index Mélanique ; UA : moyenne +/- SEM | | | | |

Ces résultats sont également illustrés dans la figure 5.

Après 30 jours d'utilisation du complément alimentaire comprenant le complexe protéomélanique bioassimilable (produit), les mesures au Mexamètre® ont montré une peau significativement plus pigmentée (augmentation de l'index mélanique) après une, deux ou trois expositions UVB/UVA (après trois expositions, augmentation de l'index mélanique de 217% comparé à avant utilisation).

### 3.2.3) Effet du complément alimentaire comprenant le complexe protéomélanique bioassimilable (produit), sur une zone non exposée Spectrocolorimètre®

Une synthèse des résultats est présentée dans le tableau 5 ci-dessous.

**Tableau 5**

| | L* | | | b* | | | ITA° | | |
|---|---|---|---|---|---|---|---|---|---|
| | Variation | | p | Variation | | p | Variation | | p |
| | UA | % | | UA | % | | UA | % | |
| J10 | 0,6 +/- 0,2 | 1 | 0,004 | 0,5 +/- 0,2 | 2 | 0,010 | 0,3 +/- 0,2 | 1 | 0,139 |
| J20 | 0,1 +/- 0,2 | 0 | 0,604 | 0,0 +/- 0,2 | 0 | 0,804 | 0,0 +/- 0,0 | 0 | 0,000 |
| J30 | 0,1 +/- 0,2 | 0 | 0,722 | -0,1 +/- 0,2 | -1 | 0,552 | 0,0 +/- 0,0 | 0 | 0,000 |
| J33 | 0,1 +/- 0,2 | 0 | 0,549 | -0,1 +/- 0,2 | 0 | 0,689 | 0,0 +/- 0,0 | 0 | 0,000 |
| J36 | 0,0 +/- 0,2 | 0 | 0,975 | -0,2 +/- 0,2 | -1 | 0,358 | 0,0 +/- 0,0 | 0 | 0,000 |
| J39 | 0,1 +/- 0,2 | 0 | 0,613 | -0,1 +/- 0,2 | 0 | 0,714 | 0,0 +/- 0,0 | 0 | 0,000 |

| | | | | | | | | | |
|---|---|---|---|---|---|---|---|---|---|
| UA : moyenne +/- SEM | | | | | | | | | |

Ces résultats sont également illustrés aux figures 6A et 6B.

Durant toute la prise du complément alimentaire comprenant le complexe protéomélanique bioassimilable (produit), les mesures au Spectrocolorimètre® n'ont pas montré de variation pertinente de la couleur de la peau sur une zone non exposée aux UV : variations de 0% à 2%, non significatives pour la plupart et non pertinentes du point de vue biologique (non visible à l'oeil nu).

### Mexamètre®

Une synthèse des résultats est présentée dans le tableau 6 ci-dessous.

**Tableau 6**

| | Variation | | p |
|---|---|---|---|
| | UA | % | |
| J10 | 3,0 +/- 1,3 | 5 | 0,027 |
| J20 | 2,7 +/- 1,4 | 5 | 0,064 |
| J30 | 3,4 +/- 1,4 | 6 | 0,020 |
| J33 | 3,3 +/- 1,2 | 6 | 0,011 |
| J36 | 2,1 +/- 1,2 | 4 | 0,088 |
| J39 | 3,1 +/- 1,2 | 5 | 0,014 |

| | | | |
|---|---|---|---|
| UA : moyenne +/- SEM | | | |

Ces résultats sont également illustrés à la figure7.

Après 10 à 39 jours d'utilisation du complément alimentaire comprenant le complexe protéomélanique bioassimilable (produit), les mesures au Mexamètre® ont montré une très légère augmentation de l'index mélanique (variation significative ou à la limite de la significativité entre 4% et 6%).

### 3.3) CONCLUSION

L'objectif principal de cette étude était d'évaluer l'effet du complément alimentaire comprenant le complexe protéomélanique bioassimilable (produit), sur la pigmentation de la peau sous stimulation UV, après 10, 20 et 30 jours d'utilisation du produit étudié.

Dans les conditions de cette étude, le complément alimentaire comprenant le complexe protéomélanique bioassimilable (produit) testé :
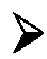 a permis d'améliorer significativement la pigmentation immédiate 2 heures après une exposition UVA. Cet effet a été observé à la fois avec le Spectrocolorimètre® et le Mexamètre®, dès 10 jours d'utilisation. L'efficacité du produit est encore augmentée après 30 jours d'utilisation.
   Une augmentation de la pigmentation de 225% a été observée avec le Spectrocolorimètre® et de 106% avec le Mexamètre®.
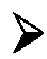 a permis d'améliorer significativement la pigmentation retardée après une, deux ou trois expositions UVB/UVA. Cet effet a été observé à la fois avec le Spectrocolorimètre® et le Mexamètre®, dès 30 jours d'utilisation du produit. Après 3 expositions UVB/UVA, une augmentation de la pigmentation de 206% a été observée avec le Spectrocolorimètre® et de 217% avec le Mexamètre®.
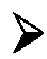 a induit une très légère augmentation de l'index mélanique sur une zone non exposée aux UV (variation de 4% à 6%). Cet effet a été observé dès 10 jours d'utilisation du produit.

Il est notable que le produit a été grandement apprécié par la majorité des sujets. 66% des sujets ont trouvé leur peau plus colorée et 76% souhaiteraient continuer l'utilisation du produit. Le produit a de plus été bien toléré par le panel de testeur. Aucune sensation d'inconfort ou d'intolérance n'a été rapportée par les sujets.

### Exemple 4 : Evaluation de l'effet d'un complexe protéomélanique bioassimilable selon l'invention sur la pigmentation des cheveux

L'objectif de cette étude est d'évaluer l'effet du complexe protéomélanique bioassimilable selon l'invention, préparé à l'exemple 1, sur la pigmentation des cheveux.

L'étude a été conduite sur 32 volontaires présentant des cheveux blancs et/ou poivre et sel et n'appliquant aucune teinture pendant la durée de l'étude.

Des mesures de la couleur des cheveux sont faites à l'aide d'un Chromamètre® à J0, J60, J90 et J120 afin d'évaluer l'effet du produit sur la pigmentation des cheveux.

### 4.1) Mesures au Chromamètre® au niveau des cheveux

A J0, les cheveux ont été rasés sur une mini-zone de 1 cm² afin de localiser parfaitement les mesures durant toute l'étude. Cette mini-zone était identifiée dans le CRF à l'aide de mesures centimétriques.

Quatre mesures colorimétriques ont été effectuées sur la **racine** des cheveux entourant la mini-zone rasée (en haut, en bas, à gauche et à droite) à l'aide d'un Chromamètre® MINOLTA CR-400, muni d'une tête de 8 mm de diamètre. L'analyse des données a été effectuée sur la moyenne des quatre mesures.

Aux temps de cinétique suivants, la mini-zone était identifiée et rasée à nouveau si besoin et les mesures au Chromamètre® comme expliqué ci-dessus.

Le Chromamètre® convertit les couleurs situées dans la plage de perception humaine en un code numérique composé de trois paramètres :
**L*** : représente la clarté (du sombre au pâle),
**a*** : représente la gamme des verts aux rouges,
**b*** : représente la gamme des bleus aux jaunes.
a* et b* sont des paramètres de chrominance et L* un paramètre de luminance.

Il devient alors possible d'exprimer, dans les moindres détails, les différences entre deux zones qui paraissent être de la même couleur. Après calibration, les mesures sont réalisées directement sur les cheveux à l'aide d'une source de lumière Xénon pulsée et d'un système à double faisceaux pour mesurer la lumière émise et corriger toute légère déviation.

Cet instrument est couramment utilisé en cosmétique et en médecine pour mesurer la couleur de la peau ou des cheveux.

### 4.2) Déroulement de l'essai

### A JO :

- Les sujets viennent au laboratoire sans avoir appliqué de produit sur les cheveux depuis la veille au soir.
- Définition et rasage d'une mini-zone de 1cm² au niveau du cuir chevelu.
- Mesures au Chromamètre® sur la racine des cheveux autour de cette mini-zone. Quatre mesures sont effectuées en haut, en bas, à gauche et à droite de la zone.

### A J60, J90, J120 :

- Les sujets viennent au laboratoire sans avoir appliqué de produit sur les cheveux depuis la veille au soir.
- Localisation de la mini-zone définie à J0. Cette zone est à nouveau rasée.
- Mesures au Chromamètre® sur la racine des cheveux autour de cette mini-zone. Quatre mesures sont effectuées en haut, en bas, à gauche et à droite de la zone.

### 4.3) Analyse des données

L'analyse statistique permet de déterminer la significativité des variations sous l'effet du produit testé.

La comparaison porte sur les valeurs obtenues aux différents temps d'évaluation.

Le test utilisé est le test t de Student sur données appariées. Les conditions d'application sont le caractère aléatoire et simple des échantillons et la normalité de la population des différences.

Le principe du test consiste à poser une hypothèse nulle (H0) d'absence de différence entre l'effet moyen aux différents temps d'évaluation (*d̅* = 0) et une hypothèse alternative H1 (notre hypothèse de recherche) d'une différence entre les temps d'évaluation (*d̅* <> 0).

On détermine ensuite quelle est la probabilité p d'observer un écart entre les temps au moins aussi grand que celui qui a été observé si l'hypothèse nulle est vraie.
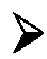 Si p < 5%, on rejette l'hypothèse nulle. On accepte alors l'hypothèse alternative H1 d'une différence significative entre les temps d'évaluation.
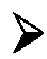 Si p > 5%, on accepte l'hypothèse nulle. Les données n'ont pas permis de mettre en évidence une différence significative entre les temps d'évaluation.

### 4.4) Résultats

### Chromamètre®

Une synthèse des résultats est présentée dans le tableau 7 ci-dessous.

**Tableau 7**

| | J60 | | | J90 | | | J120 | | |
|---|---|---|---|---|---|---|---|---|---|
| | Variation | | p | Variation | | p | Variation | | p |
| | U.A. | % | | U.A. | % | | U.A. | % | |
| L* | -2,2 +/- 0,3 | -4 | <0,001 | -4,9 +/- 0,4 | -9 | <0,001 | -7,5 +/- 0,5 | -13 | <0,001 |
| a* | -0,5 +/- 0,2 | -8 | 0,012 | -1,4 +/- 0,2 | -22 | <0,001 | -2,4 +/- 0,2 | -37 | <0,001 |
| b* | -0,6 +/- 0,1 | -6 | <0,001 | -1,3 +/- 0,2 | -14 | <0,001 | -2,0 +/- 0,2 | -22 | <0,001 |

| | | | | | | | | | |
|---|---|---|---|---|---|---|---|---|---|
| UA : moyenne +/- SEM | | | | | | | | | |

Ces résultats sont également illustrés à la figure 8.
Après 30, 90 et 120 jours d'utilisation du complément alimentaire comprenant le complexe protéomélanique bioassimilable (produit), les mesures au Chromamètre® ont montré une couleur des cheveux significativement plus sombre (13% après 120 jours d'utilisation, p<0,001). L'effet augmente avec le temps de traitement. Une variation de la teinte des cheveux a également été observée avec une couleur moins "jaune" et moins "rouge".

### Exemple 5 : Etude de la bioassimilabilité du complexe protéomélanique selon l'invention.

### Système expérimental

L'évaluation de la bioassimilabilité du complexe protéomélanique selon l'invention et des différents composants dudit complexe dans les fluides gastriques et intestinaux simulés a été étudié en suivant la procédure de tube de dialyse [D.W. BOLLINGER et coll. ; J. Agric. Food Chem. ; 2005. 53 : 3287-3294].

### Digestion à la pepsine.

Une quantité de 100 mg de complexe mélanoprotéique selon l'invention a été mélangé avec 1,0 mL d'une solution 0,85 N d'acide chlorhydrique (HCl).

24 000 U de pepsine porcine par mL ont été ensuite additionnés et l'échantillon a été incubé dans un bain d'eau à 39 °C pendant 120 min.

### Digestion pancréatine.

A la fin de la digestion à la pepsine, les échantillons ont été transférés dans des tubes de dialyse de 18 cm de long et 1,3 mL d'une solution 0,8 M de NaHC0₃ contenant 22,60 mg porcine pancréatine/mL (8 x USP) ont été ajoutés au digesta de pepsine. Les tubes de dialyse ont été scellés à chaque extrémité avec des pinces. Les tubes de dialyse de porosité supérieure à 12000 et de diamètre de 1,6 cm (Sigma Chemical Co., référence D6191) ont été placés dans un ballon de 250 mL contenant 100 mL de tampon 0,05 M de succinate. Les échantillons ont été incubés à 39 °C sous agitation à 120 cycles par minute pendant 4 h.

Après incubation de la pancréatine, les composants disponibles dans le milieu de dialyse ont été déterminés par différentes procédures expérimentales.

Toutes les expériences ont été réalisées en triplicate et les résultats sont exprimés comme la moyenne des résultats des 3 expériences.

### Détermination quantitative des protéines par la méthode de Lowry

1 mL de chaque échantillon a été mélangé avec 4,5 mL de réactif de Lowry (9,8 mL de Na₂C0₃ (2% de P/V), 0,1 mL de CuS0₄-5H₂0 (1% P/V) et 0,1 mL de tartrate Na⁺-K⁺ (0,5% P/V). La solution a été mélangée et incubée pendant 10 min. Puis 0,5 mL de réactif de Folin-Ciocalteau 1N ont été ajoutés et laissés réagir pendant 30 min. Enfin, l'absorbance a été mesurée à 660 nm [O.H. LOWRY et coll. ; J. Biol. Chem. ; 1951. 193: 265-275].

Les données sont exprimées en pourcentage de protéines disponibles.

### Détermination quantitative de la tyrosine par HPLC

Le système HPLC consistait en une pompe Jasco BIP-je, un injecteur Rheodyne 7725 (boucle de 230 µL), et un détecteur UV Jasco UVDEC-100 V.

Une colonne de type C8 (0,4 x 15 mm) garnie de particules de 5 µm de taille a été utilisé pour la détermination. La séparation a été contrôlée à 210 nm. Une phase mobile constituée d'un mélange acétonitrile-eau de mélange (5:95 v/v) a été utilisée. Le débit a été maintenu à 1,5 mL min₋₁

L'identification des pics a été effectuée en comparant le temps de rétention d'échantillons avec une solution étalon de tyrosine.

Les données sont exprimées en pourcentage de tyrosine disponible.

### Détermination quantitative de mélanine par méthodes spectrophotométriques

Après les incubations de pepsine et de pancréatine, la quantité de mélanine disponible a été déterminée par spectrophotométrie à l'aide d'un spectromètre Jasco V-530 UV/Vis, selon la méthode développée par Ozeki et ses collaborateurs [Ozeki, H., et coll. ; 1996. Spectrophotometric characterization of eumelanin and pheomelanin in hair. Pigment Cell Research 9:265-270 ou Ozeki, H.et coll. ; 1995. Chemical characterization of hair melanins in various coat-color mutants of mice. Journal of Investigative Dermatology 105:361-366], méthode légèrement modifiée par les inventeurs.

Les échantillons obtenus à la suite des digestions à la pepsine et à la pancréatine ont été séchés sous vide et le résidu a été redissous dans du Soluène-350 (Perkin Elmer), une base organique forte formulé avec du toluène. Les échantillons ont été analysés pour déterminer les absorbances à 500 nm (A500). Les valeurs A500 correspondent à la mélanine totale contenue dans l'échantillon.

Les concentrations totales de mélanine ont été calculées par référence à une courbe d'étalonnage établie à partir de cinq solutions standard de mélanine différentes (0,02, 0,04, 0,06, 0,08 et 0,10 mg/ml) dans du Soluène-350 (voir Figure 10). L'absorbance des solutions standard a été mesurée à 500 nm pour établir une courbe d'étalonnage et le coefficient de corrélation (R2), pente et l'ordonnée de l'équation de régression obtenue ont été calculés par la méthode des moindres carrés.

Les valeurs d'absorbance des échantillons mélaniques obtenus par les études de biodisponibilité in vitro effectuées en triple, et les pourcentages de biodisponibilité correspondant, ont été rapportés dans le tableau 8 ci-dessous.

**Tableau 8.**

| Abs | Biodisponibilité (%) |
|---|---|
| 0,7535 | 8,92 |
| 0,7832 | 9,31 |
| 0,8526 | 10,06 |

La valeur finale en vitro de la biodisponibilité (9,43 ± 0,68%) a été calculée comme la moyenne des données obtenues par les trois expériences effectuées (Tableau 1)

Les données ont été exprimées en pourcentage de mélanine disponible.

### RÉSULTATS

Les résultats sont présentés à la figure 9

La méthode des tubes de dialyse est une méthode rapide et de faible coût pour évaluer la bioassimilabilité de différents types de composés.

Comme précédemment expliqué, les composants du complexe protéomélanique sont déterminés en utilisant différentes procédures expérimentales.

Plus précisément, pour la quantification de la teneur en protéines dans le digesta gastro-intestinal, le dosage de Lowry a été utilisé.

Une bioassimilabilité des protéines dans les liquides gastro-intestinaux simulés de 40,5 ± 0,8% a été mesurée.

La quantification de la tyrosine a été réalisée par analyse HPLC en comparant les chromatogrammes des échantillons à celle d'une solution standard de tyrosine.

Une bioassimilabilité égale à 1,85 ± 0,2% a été ainsi déterminée.

Enfin, la bioassimilabilité de la mélanine dans les fluides gastro-intestinaux simulés a été déterminée par analyse spectrophotométrique.

Une bioassimilabilité égale à 9,43 ± 0,7% a ainsi été déterminée.

La bioassimilabilité totale du complexe protéomélanique a été mathématiquement déterminée en ajoutant la teneur en protéines, en tyrosine et en mélanine; il en résulte une valeur de la bioassimilabilité du complexe protéomélanique de 49,93 (figure 9).

### Exemple 5 : Evaluation de l'effet d'un complexe protéomélanique bioassimilable selon la cohésivité cellulaire dans la peau humaine.

La mesure de la teneur en protéine de la peau permet d'évaluer la cohésivité des cellules.

L'évaluation de la cohésivité de la peau en fonction de sa teneur en protéines est utile pour évaluer l'efficacité de la cohésivité cellulaire dû au traitement reçu par les sujets de l'étude. Une diminution de la quantité de protéines exsudées à la surface de la peau reflète une augmentation de la cohésivité cellulaire.

La teneur en protéines (cohésivité) est mesurée en utilisant la méthode de la feuille Corneofix à l'aide du kit Corneofix® F 20 (Courage + Khazaka electronic GmbH), selon le protocole du fournisseur. Les échantillons non invasifs de 10 couches de la couche cornée d'un visage propre ont ainsi été obtenus pour déterminer la teneur en protéines.

La méthode de Lowry (Oliver H. Lowry, Nira J. Rosebrough, A Lewis Farr et Rose J. Randall, « Protein measurement with the Folin phenol reagent », J. biol. Chem., vol. 193, n° 1, 1951, p. 265-275) est utilisée pour mesurer la teneur en protéines. Elle est basée sur la capacité du cuivre à se lier à des protéines dans des conditions alcalines, et quand le réactif de Folin est ajouté, il se forme un complexe avec la protéine qui peut être vue à 550 nm.

Les sujets sont répartis en 2 groupes, un groupe témoin et un groupe dit "actif".

Le groupe "actif" [■] a reçu 500 mg/jour en 1 prise de complexe mélanoprotéique selon l'invention pendant 90 jours.

Le groupe "Témoin" [□] a reçu 500 mg/jour en 1 prise de maltodextrine pendant 90 jours.

Les sujets du groupe témoin (n'ayant pas reçu ce complexe protéomélanique selon l'invention), n'ont montré aucune amélioration de la teneur en protéines de la peau à partir de la ligne de base au jour 90. Les sujets ayant reçu du complexe protéomélanique selon l'invention, ont amélioré leur teneur en protéines de la peau de manière significative par rapport à la ligne de base au jour 90. On note jusqu'à moins de 15,9% de protéines captées dans les feuilles Corneofix® F 20 (P <0,001).

La prise de complexe protéomélanique a également significativement amélioré la teneur en protéines de la peau par rapport au témoin au jour 90 avec une différence de 19,7% par rapport au témoin (P <0,001).

95,8% des sujets du groupe actif ont montré une amélioration de la cohésivité de la peau.

Les résultats de cette étude sont présentés à la figure 11.

## Revendications

1. Complexe protéomélanique bioassimilable, comprenant au moins un extrait protéique, soluble ou partiellement soluble et de la mélanine, ledit complexe protéomélanique étant bioactif et ledit extrait protéique comprenant au moins un résidu cystéine ou au moins un résidu tyrosine ou au moins un résidu cystéine et un résidu tyrosine **caractérisé en ce que** les protéines et/ou les fragments de protéines dudit extrait protéique ont été rendus solubles par greffage d'au moins un groupement sulfite sur au moins un atome de soufre d'au moins une cystéine desdites protéines et/ou desdits fragments de protéines dudit extrait protéique.

2. Complexe protéomélanique selon la revendication 1, **caractérisée en ce que** ledit extrait protéique est soluble dans l'eau de 0,1 à 99 %, préférentiellement de 0,1 à 75%, très préférentiellement de 0,1 à 50%.

3. Complexe protéomélanique selon l'une quelconque des revendications 1 ou 2, **caractérisée en ce que** qu'il comprend entre 0,01% à 99,99% d'extrait protéique, préférentiellement entre 1% et 95%, très préférentiellement entre 30% et 95%, encore plus préférentiellement entre 50% et 95%.

4. Complexe protéomélanique selon l'une quelconque des revendications 1 à 3, **caractérisée en ce qu'**il comprend entre 0,01% à 99,99% de mélanine, préférentiellement entre 0,5% et 20%, très préférentiellement entre 1% et 15%.

5. Complexe protéomélanique selon l'une quelconque des revendications 1 à 4, **caractérisée en ce que** l'extrait protéique comprend des peptides de longueur comprise entre 2 et 1000 acides aminés, préférentiellement entre 2 et 500 acides aminés, très préférentiellement entre 2 et 100 acides aminés.

6. Complexe protéomélanique selon l'une quelconque des revendications 1 à 5, **caractérisée en ce que** ledit extrait protéique et/ou ladite mélanine sont issus de sources protéiques et mélaniques différentes ou identiques, prises séparément ou sont issus d'une même source mélanoprotéique, avantageusement utilisée seule.

7. Complexe protéomélanique selon l'une quelconque des revendications 1 à 6, **caractérisée en ce que** ledit extrait protéique et/ou ladite mélanine sont issus de laine, de poils, de cheveux, de griffes, de corne ou encore de plumes, de plantes, de fruits, d'encre de céphalopodes, de bactéries ou de sources synthétiques préférentiellement issus de laine.

8. Complexe protéomélanique selon l'une quelconque des revendications 1 à 7, **caractérisée en ce que** ledit extrait protéique et/ou ladite mélanine sont issus de laine de moutons, de mouflons, de chèvres, de chamois, de takin, de bouquetins, de yanghirs, de thars, de jharal, de serows, de goral, de boeuf musqué, d'urial, de bharal, d'isard, de lapins, de lièvres, de pikas, de lama, d'alpaga, de guanaco, de vigogne, de chameau, de dromadaire, de yack ou de plumes comme celle de pie, de corbeau ou de merle, préférentiellement de la laine de mouton, très préférentiellement de mouton noir ("mouton d'Ouessant", "Noire du Velay", *"Nez-noir* du Valais", *"mouton Noir* de Thibar", "Black Welsh Mountain Sheep", "Balwen", "Zwartbles" ou encore "Hebridean").

9. Procédé de préparation d'un complexe protéomélanique bioassimilable tel que décrit à l'une quelconque des revendications 1 à 8 dans lequel dans une première étape on rend la partie protéique de ladite source mélanoprotéique solubilisable, avantageusement hydrosolubilisable, par greffage d'au moins un groupement sulfite sur l'atome de soufre des cystéines desdites protéines et/ou fragments de protéines (peptides), et dans une deuxième étape on fractionne la partie protéique du mélange obtenu à la première étape en peptides, pour obtenir un mélange comprenant le complexe protéomélanique recherché.

10. Procédé de préparation d'un complexe protéomélanique bioassimilable selon la revendication 9, **caractérisé en ce qu'**il comprend en outre une troisième étape dans laquelle on ajoute de la mélanine au mélange obtenu à la deuxième étape pour obtenir un mélange comprenant le complexe protéomélanique recherché.

11. Procédé selon la revendication 9, **caractérisé en ce que** le greffage d'un sulfite sur l'atome de soufre des cystéines est réalisé par S-sulfonation, avantageusement par sulfitolyse oxydative.

12. Procédé selon l'une quelconque des revendications 9 à 11, **caractérisé en ce que** la deuxième étape d'hydrolyse est réalisée par hydrolyse acide, hydrolyse basique, hydrolyse enzymatique, ou encore par oxydation partielle en utilisant de l'acide peracétique, du peroxyde d'hydrogène ou un équivalent, préférentiellement par hydrolyse enzymatique.

13. Complexe protéomélanique bioassimilable tel que décrit dans les revendications 1 à 8 pour stimuler la mélanogénèse.

14. Complexe protéomélanique bioassimilable selon la revendication 13, pour stimuler la mélanogénèse dans la peau, les cheveux, les poils, la laine, les phanères, le cerveau, le canal intérieur de l'oreille, et/ou les yeux.

15. Complexe protéomélanique bioassimilable tel que décrit dans les revendications 1 à 8, pour protéger les organismes vivants des rayonnements ionisants ou non.

16. Complexe protéomélanique bioassimilable tel que décrit dans les revendications 1 à 8, pour protéger les organismes vivants du rayonnement ultraviolet, lointain ou proche (A, B ou C), de la lumière visible, du rayonnement Infrarouge (A, B et/ou C) ou encore des Rayons X.

17. Complexe protéomélanique bioassimilable tel que décrit dans les revendications 1 à 8 pour piéger les radicaux libres.

18. Complexe protéomélanique bioassimilable tel que décrit dans les revendications 1 à 8 pour combattre le vieillissement tissulaire.

19. Complexe protéomélanique bioassimilable tel que décrit dans les revendications 1 à 8, pour augmenter la régénération cellulaire, l'élasticité et/ou l'hydratation de la peau, pour renforcer la cohésion cellulaire, particulièrement dans la peau et/ou les phanères.

20. Complexe protéomélanique bioassimilable tel que décrit dans les revendications 1 à 8 pour combattre, les rides et les ridules de la peau.

21. Complexe protéomélanique bioassimilable tel que décrit dans les revendications 1 à 8 pour lutter contre le stress oxydatif et traiter ou prévenir ses conséquences.

22. Complexe protéomélanique bioassimilable tel que décrit dans les revendications 1 à 8, pour piéger les métaux lourds, particulièrement dans l'organisme humain.

23. Complexe protéomélanique bioassimilable tel que décrit dans les revendications 1 à 8, pour prévenir et/ou traiter la maculopathie liée à l'âge (MLA) et/ou la dégénération maculaire liée à l'âge (DMLA).

24. Complexe protéomélanique bioassimilable tel que décrit dans les revendications 1 à 8, **caractérisé en ce qu'**il est sous toute forme galénique connue, particulièrement sous la forme d'une poudre, d'un liquide, d'une crème, d'une lotion, d'un patch ou encore d'un spray.

25. Utilisation d'au moins un complexe protéomélanique bioassimilable tel que décrit dans les revendications 1 à 8 ou 13 à 24, pour la préparation de compositions cosmétique, dermatologique, pharmaceutique ou encore alimentaire.

26. Composition, avantageusement cosmétique, dermatologique, pharmaceutique ou encore alimentaire, comprenant au moins un complexe protéomélanique bioassimilable tel que décrit dans les revendications 1 à 8 ou 13 à 24.

## Patentansprüche

1. Bioassimilierbarer Protein-Melanin-Komplex, der wenigstens einen löslichen oder teilweise löslichen Proteinextrakt und Melanin umfasst, wobei der genannte Protein-Melanin-Komplex bioaktiv ist und der genannte Proteinextrakt wenigstens einen Cysteinrest oder wenigstens einen Tyrosinrest oder wenigstens einen Cysteinrest und einen Tyrosinrest umfasst, **dadurch gekennzeichnet, dass** die Proteine und/oder die Proteinfragmente des genannten Proteinextrakts durch Pfropfen von wenigstens einer Sulfitgruppe auf wenigstens ein Schwefelatom von wenigstens einem Cystein der genannten Proteine und/oder der genannten Proteinfragmente des genannten Proteinextrakts löslich gemacht wurden.

2. Protein-Melanin-Komplex nach Anspruch 1, **dadurch gekennzeichnet, dass** der genannte Proteinextrakt in Wasser zu 0,1 bis 99 %, vorzugsweise zu 0,1 bis 75 %, sehr bevorzugt zu 0,1 bis 50 % löslich ist.

3. Protein-Melanin-Komplex nach einem der Ansprüche 1 oder 2, **dadurch gekennzeichnet, dass** er zwischen 0,01 % und 99,99 % Proteinextrakt umfasst, vorzugsweise zwischen 1 % und 95 %, sehr bevorzugt zwischen 30 % und 95 %, noch bevorzugter zwischen 50 % und 95 %.

4. Protein-Melanin-Komplex nach einem der Ansprüche 1 bis 3, **dadurch gekennzeichnet, dass** er zwischen 0,01 % und 99,99 % Melanin umfasst, vorzugsweise zwischen 0,5 % und 20 %, sehr bevorzugt zwischen 1 % und 15 %.

5. Protein-Melanin-Komplex nach einem der Ansprüche 1 bis 4, **dadurch gekennzeichnet, dass** der Proteinextrakt Peptide mit einer Länge zwischen 2 und 1000 Aminosäuren, vorzugsweise zwischen 2 und 500 Aminosäuren, sehr bevorzugt zwischen 2 und 100 Aminosäuren umfasst.

6. Protein-Melanin-Komplex nach einem der Ansprüche 1 bis 5, **dadurch gekennzeichnet, dass** der genannte Proteinextrakt und/oder das genannte Melanin von verschiedenen oder identischen Protein- und Melaninquellen stammt/stammen, getrennt genommen, oder von ein und derselben Melanoproteinquelle stammt/stammen, die vorteilhafterweise allein verwendet wird.

7. Protein-Melanin-Komplex nach einem der Ansprüche 1 bis 6, **dadurch gekennzeichnet, dass** der genannte Proteinextrakt und/oder das genannte Melanin von Wolle, Borsten, Haaren, Krallen, Hörnern oder auch Federn, Pflanzen, Früchten, der Tinte von Kopffüßern, Bakterien oder synthetischen Quellen, vorzugsweise von Wolle, stammt/stammen.

8. Protein-Melanin-Komplex nach einem der Ansprüche 1 bis 7, **dadurch gekennzeichnet, dass** der genannte Proteinextrakt und/oder das genannte Melanin von der Wolle von Schafen, Mufflons, Ziegen, Gämsen, Takinen, Steinböcken, sibirischen Steinböcken, Tahrs, Himalaya-Tahrs, Serauen, Goralen, Moschusochsen, Urialen, Bharalen, Pyrenäen-Gämsen, Kaninchen, Hasen, Pikas, Lamas, Alpakas, Guanakos, Vikunjas, Kamelen, Dromedaren, Yaks oder von Federn wie denen von Elster, Krähe oder Amsel, vorzugsweise von Schafwolle, sehr bevorzugt von schwarzen Schafen ("Ouessant", "Noire du Velay", "Walliser Schwarznasenschaf", "*Noir* de Thibar", "Black Welsh Mountain", "Balwen", "Zwartbles" oder auch "Hebriden"-Schafen) stammt/stammen.

9. Verfahren zur Herstellung eines bioassimilierbaren Protein-Melanin-Komplexes nach einem der Ansprüche 1 bis 8, bei dem in einem ersten Schritt der Proteinteil der genannten Melanoproteinquelle durch Pfropfen von wenigstens einer Sulfitgruppe auf das Schwefelatom der Cysteine der genannten Proteine und/oder Proteinfragmente (Peptide) löslich, vorteilhafterweise wasserlöslich, gemacht wird und in einem zweiten Schritt der Proteinteil des im ersten Schritt erhaltenen Gemischs zu Peptiden fraktioniert wird, um ein Gemisch zu erhalten, das den angestrebten Protein-Melanin-Komplex umfasst.

10. Verfahren zur Herstellung eines bioassimilierbaren Protein-Melanin-Komplexes nach Anspruch 9, **dadurch gekennzeichnet, dass** es ferner einen dritten Schritt beinhaltet, bei dem Melanin zu dem im zweiten Schritt erhaltenen Gemisch gegeben wird, um ein Gemisch zu erhalten, das den angestrebten Protein-Melanin-Komplex umfasst.

11. Verfahren nach Anspruch 9, **dadurch gekennzeichnet, dass** das Pfropfen eines Sulfits auf das Schwefelatom der Cysteine durch S-Sulfonierung, vorteilhafterweise durch oxidative Sulfitolyse, durchgeführt wird.

12. Verfahren nach einem der Ansprüche 9 bis 11, **dadurch gekennzeichnet, dass** der zweite Schritt der Hydrolyse durch saure Hydrolyse, basische Hydrolyse, enzymatische Hydrolyse oder auch durch partielle Oxidation mit Peressigsäure, Wasserstoffperoxid oder einem Äquivalent, vorzugsweise durch enzymatische Hydrolyse, durchgeführt wird.

13. Bioassimilierbarer Protein-Melanin-Komplex nach Anspruch 1 bis 8 zur Stimulierung der Melanogenese.

14. Bioassimilierbarer Protein-Melanin-Komplex nach Anspruch 13 zur Stimulierung der Melanogenese in der Haut, den Haaren, den Borsten, der Wolle, den keratinisierten Gliedmaßen, dem Gehirn, dem Innenohrgang und/oder den Augen.

15. Bioassimilierbarer Protein-Melanin-Komplex nach Anspruch 1 bis 8 zum Schutz lebender Organismen vor ionisierender oder nichtionisierender Strahlung.

16. Bioassimilierbarer Protein-Melanin-Komplex nach Anspruch 1 bis 8 zum Schutz lebender Organismen vor ferner oder naher ultravioletter Strahlung (A, B oder C), sichtbarem Licht, Infrarotstrahlung (A, B und/oder C) oder auch Röntgenstrahlen.

17. Bioassimilierbarer Protein-Melanin-Komplex nach Anspruch 1 bis 8 zum Einfangen freier Radikale.

18. Bioassimilierbarer Protein-Melanin-Komplex nach Anspruch 1 bis 8 zur Bekämpfung der Gewebealterung.

19. Bioassimilierbarer Protein-Melanin-Komplex nach Anspruch 1 bis 8 zur Erhöhung der Zellregeneration, der Elastizität und/oder der Hydrierung der Haut, zur Stärkung der Zellkohäsion, insbesondere in der Haut und/oder den keratinisierten Gliedmaßen.

20. Bioassimilierbarer Protein-Melanin-Komplex nach Anspruch 1 bis 8 zur Bekämpfung von Falten und feinen Linien in der Haut.

21. Bioassimilierbarer Protein-Melanin-Komplex nach Anspruch 1 bis 8 zur Bekämpfung von oxidativem Stress und Behandlung oder Verhütung seiner Folgen.

22. Bioassimilierbarer Protein-Melanin-Komplex nach Anspruch 1 bis 8 zum Einfangen von Schwermetallen, insbesondere im menschlichen Organismus.

23. Bioassimilierbarer Protein-Melanin-Komplex nach Anspruch 1 bis 8 zur Verhütung und/oder Behandlung von altersbedingter Makulopathie (ARM) und/oder altersbedingter Makuladegeneration (ARMD).

24. Bioassimilierbarer Protein-Melanin-Komplex nach Anspruch 1 bis 8, **dadurch gekennzeichnet, dass** er in einer beliebigen bekannten galenischen Form vorliegt, insbesondere in Form eines Pulvers, einer Flüssigkeit, einer Creme, einer Lotion, eines Pflasters oder auch eines Sprays.

25. Verwendung von wenigstens einem bioassimilierbaren Protein-Melanin-Komplex nach Anspruch 1 bis 8 oder 13 bis 24 zur Herstellung von kosmetischen, dermatologischen, pharmazeutischen oder auch lebensmitteltechnischen Zusammensetzungen.

26. Zusammensetzung, vorteilhafterweise kosmetisch, dermatologisch, pharmazeutisch oder auch lebensmitteltechnisch, die wenigstens einen bioassimilierbaren Protein-Melanin-Komplex nach Anspruch 1 bis 8 oder 13 bis 24 umfasst.

## Claims

1. Bioassimilable protein-melanin complex, comprising at least one protein extract, soluble or partially soluble, and melanin, said protein-melanin complex being bioactive and said protein extract comprising at least one cysteine residue or at least one tyrosine residue or at least one cysteine residue and one tyrosine residue, **characterized in that** the proteins and/or the protein fragments of said protein extract have been rendered soluble by grafting at least one sulphite group onto at least one sulphur atom of at least one cysteine of said proteins and/or of said protein fragments of said protein extract.

2. Protein-melanin complex according to claim 1, **characterized in that** said protein extract is soluble in water from 0.1 to 99%, preferentially from 0.1 to 75%, very preferentially from 0.1 to 50%.

3. Protein-melanin complex according to any one of claims 1 or 2, **characterized in that** it comprises between 0.01% and 99.99% of protein extract, preferentially between 1% and 95%, very preferentially between 30% and 95%, yet more preferentially between 50% and 95%.

4. Protein-melanin complex according to any one of claims 1 to 3, **characterized in that** it comprises between 0.01% and 99.99% of melanin, preferentially between 0.5% and 20%, very preferentially between 1% and 15%.

5. Protein-melanin complex according to any one of claims 1 to 4, **characterized in that** the protein extract comprises peptides with a length comprised between 2 and 1,000 amino acids, preferentially between 2 and 500 amino acids, very preferentially between 2 and 100 amino acids.

6. Protein-melanin complex according to any one of claims 1 to 5, **characterized in that** said protein extract and/or said melanin originate(s) from different or identical protein and melanin sources, taken separately or originate(s) from one and the same melanoprotein source, advantageously used alone.

7. Protein-melanin complex according to any one of claims 1 to 6, **characterized in that** said protein extract and/or said melanin originate(s) from wool, bristles, hair, claws, horns or also feathers, plants, fruits, the ink from cephalopods, bacteria or synthetic sources, preferentially originating from wool.

8. Protein-melanin complex according to any one of claims 1 to 7, **characterized in that** said protein extract and/or said melanin originate(s) from the wool of sheep, mouflons, goats, chamois, takin, ibexes, Siberian ibexes, thars, Himalayan thars, serows, goral, musk ox, urial, bharal, isard, rabbits, hares, pikas, llama, alpaca, guanaco, vicuna, camel, dromedary, yak or feathers such as those from magpie, crow or blackbird, preferentially from the wool of sheep, very preferentially from black sheep ("Ouessant", "Noire du Velay", "Valais Blacknose", *"Noir* de Thibar", "Black Welsh Mountain", "Balwen", "Zwartbles" or also "Hebridean" sheep).

9. Process for the preparation of a bioassimilable protein-melanin complex as described in any one of claims 1 to 8 in which in a first step the protein part of said melanoprotein source is rendered solubilizable, advantageously water-solubilizable, by grafting at least one sulphite group onto the sulphur atom of the cysteines of said proteins and/or protein fragments (peptides), and in a second step the protein part of the mixture obtained in the first step is fractionated to peptides, in order to obtain a mixture comprising the sought protein-melanin complex.

10. Process for the preparation of a bioassimilable protein-melanin complex according to claim 9, **characterized in that** it comprises moreover a third step in which melanin is added to the mixture obtained in the second step in order to obtain a mixture comprising the sought protein-melanin complex.

11. Process according to claim 9, **characterized in that** the grafting of a sulphite onto the sulphur atom of the cysteines is carried out by S-sulphonation, advantageously by oxidative sulphitolysis.

12. Process according to any one of claims 9 to 11, **characterized in that** the second step of hydrolysis is carried out by acid hydrolysis, basic hydrolysis, enzymatic hydrolysis, or also by partial oxidation using peracetic acid, hydrogen peroxide or an equivalent, preferentially by enzymatic hydrolysis.

13. Bioassimilable protein-melanin complex as described in claims 1 to 8 for stimulating melanogenesis.

14. Bioassimilable protein-melanin complex according to claim 13, for stimulating melanogenesis in the skin, the hair, the bristles, the wool, the keratinized appendages, the brain, the inner ear canal, and/or the eyes.

15. Bioassimilable protein-melanin complex as described in claims 1 to 8, for protecting living organisms from ionising or non-ionising radiation.

16. Bioassimilable protein-melanin complex as described in claims 1 to 8, for protecting living organisms from far or near ultraviolet radiation (A, B or C), visible light, infrared radiation (A, B and/or C) or also X-rays.

17. Bioassimilable protein-melanin complex as described in claims 1 to 8, for trapping free radicals.

18. Bioassimilable protein-melanin complex as described in claims 1 to 8, for combating tissue aging.

19. Bioassimilable protein-melanin complex as described in claims 1 to 8, for increasing cell regeneration, the elasticity and/or the hydration of the skin, for reinforcing cell cohesion, particularly in the skin and/or the keratinized appendages.

20. Bioassimilable protein-melanin complex as described in claims 1 to 8, for combating wrinkles and fine lines in the skin.

21. Bioassimilable protein-melanin complex as described in claims 1 to 8, for combating oxidative stress and treating or preventing its consequences.

22. Bioassimilable protein-melanin complex as described in claims 1 to 8, for trapping heavy metals, particularly in the human organism.

23. Bioassimilable protein-melanin complex as described in claims 1 to 8, for preventing and/or treating age-related maculopathy (ARM) and/or age-related macular degeneration (ARMD).

24. Bioassimilable protein-melanin complex as described in claims 1 to 8, **characterized in that** it is in any known galenic form, particularly in the form of a powder, a liquid, a cream, a lotion, a patch or also a spray.

25. Use of at least one bioassimilable protein-melanin complex as described in claims 1 to 8 or 13 to 24, for the preparation of cosmetic, dermatological, pharmaceutical or also food compositions.

26. Composition, advantageously cosmetic, dermatological, pharmaceutical or also food, comprising at least one bioassimilable protein-melanin complex as described in claims 1 to 8 or 13 to 24.
